# EUROPEAN PATENT SPECIFICATION

(11) **EP 3 487 481 B1**
(45) Date of publication and mention of the grant of the patent: **21.02.2024**
(21) Application number: 17749616.3
(22) Date of filing: 18.07.2017
(51) Int. Cl.: A61K 9/08, A61K 47/36, A61K 31/196, A61K 31/58, A61K 31/407, A61K 31/5415, A61K 47/69, A61P 19/02, C08B 37/16, C08L 5/16

(54) **COMPOSITIONS COMPRISING A POLYSACCHARIDE MATRIX FOR THE CONTROLLED RELEASE OF ACTIVE INGREDIENTS**
ZUSAMMENSETZUNGEN MIT EINER POLYSACCHARIDMATRIX ZUR KONTROLLIERTEN FREISETZUNG VON WIRKSTOFFEN
COMPOSITIONS COMPRENANT UNE MATRICE DE POLYSACCHARIDE POUR LA LIBÉRATION CONTRÔLÉE DE SUBSTANCES ACTIVES

(30) Priority: 19.07.2016 IT 201600075246
(43) Date of publication of application: 29.05.2019
(73) Proprietor: Jointherapeutics S.r.l., 22100 Como (IT)
(72) Inventor: MORPURGO, Margherita, 35137 Padova (IT); BIANCHINI, Giulio, 30020 Fossalta Di Piave (IT); CALLEGARO, Lanfranco, 35139 Padova (IT)
(74) Representative: Villa, Livia
(86) International application number: PCT/EP2017/068085
(87) International publication number: WO 2018/015364

(56) References cited:
- WO-A1-2007/135116
- CN-A- 104 971 039
- Hyun Choi Kyu ET AL: "Hyaluronic acid gel formulation containing Piroxicam : Hydroxypropyl-[beta]-cyclodextrin inclusion complex for intra-articular delivery", Biomaterials Research, vol. 13, no. 1 26 November 2008 (2008-11-26), 2009, pages 1-6, XP055202936, Retrieved from the Internet: URL:http://www.ksbm.or.kr/pub/13-1(1).pdf [retrieved on 2015-07-16]

## Description

### Field of the Invention

The invention relates to novel compositions comprising a clathrate consisting in a cyclodextrin and an active ingredient homogeneously dispersed in an aqueous solution of a polysaccharide polymer matrix, and their use in diseases, such as muscle-skeletal disorders characterized by an inflammatory state, wherein a combination of pharmacological and viscosupplementant actions are required.

### Background

Osteoarthritis is nowadays recognized as a pathology of the entire articulation involving all its tissues, such as cartilage, bone, ligaments, meniscus, articular capsule, synovial membrane, muscles and nervous tissue, and generally characterized by a symptomatology comprising pain, numbness, stiffness, loss of flexibility, irritation, and formation of bone spurs (Le Graverand-Gastineau M-PH et al., Curr Drug Targets, 2010, 5, 528-35; Felson DT et al., Arthritis Reum 2004, 50(2), 341-4). Among the various risk factors associated with this disease there are gender, age, obesity, genetic predisposition, joint mechanics, metabolic factors, and acute joint traumas; often the different types of osteoarthritis are linked to the various risk factors involved in the development and progression of the disease (Wieland HA et al, Nat Rev Drug Discov 2005, 4(4), 331-44; Bay-Jensen A-C et al., Rheumatol Int 2010, 30(4), 435-42). For example, in cases of acute traumatic events, responsible for about 12% of osteoarthritis, there is an increase in the level of inflammatory cytokine (IL-1, IL-6, TNF-α) in the synovial fluid, resulting in potential diffusion in the cartilage where they can trigger proteolysis and cause loss of cartilage matrix (Irie K et al., Knee 2003, 10(1), 93-6; Kapoor M et al., Nat Rev Rheumatol 2011, 7, 33-42). To date, although many active ingredients are available to modify the course of rheumatic disease (DMARDs), they are not equally available to block or reverse the course of osteoarthritis (DMOADs) (Le Graverand-Gastineau M-PH et al., Curr Drug Targets 2010, 5, 528-35; Hunter DJ, Nat Rev Rheumatol 2011, 7, 13-22; Matthews GL et al., Expert Opin Emerg Drugs 2011, 16(3), 479-91).

In this respect, it should be remembered that several active ingredients with anti-catabolic and pro-anabolic functions, such as glucocorticoids, have been identified, which have been found useful in the prevention and treatment of cartilage matrix loss associated with post-traumatic osteoarthritis (PTOA) (Hunter DJ, Nat Rev Rheumatol 2011, 7, 13-22; Lu YC et al., Arthritis Res Ther 2011, 13(5), R142; Nixon AJ et al., Clin Orthop Relat Res 2000, (Suppl. 379), S201-13; Miller RE et al., Arthritis Rheum 2010, 62(12), 3686-94). However, none of the candidates met the safety/efficacy criteria and, in particular, the incidence and the severity of systemic side effects proved to be decisive for the failure of numerous clinical trials (Matthews GL et al., Expert Opin Emerg Drugs 2011, 16(3), 479-91). Despite the lack of a pharmacological therapy able to block, and possibly reverse the course of the disease, the osteoarthritis treatment is currently aimed at improving the symptoms, and the commonly prescribed treatments consist of administering analgesic drugs, such as paracetamol, steroids (corticosteroids) and non-steroidal anti-inflammatory drugs (NSAIDs), and opioids. The pharmacological treatment with NSAIDs is one of the most commonly used, and it allows to obtain a statistically significant analgesic effect. Despite this, the use of this type of drugs is associated with several side effects, such as gastrointestinal complications, cardiovascular risk, and renal toxicity (Kennedy S et al., BC Medical Journal 2010, 52, 404-09). The use of anti-inflammatory and immunosuppressant steroid drugs is definitely another main treatment, and their on-site delivery in the joint results in good short-term pain relief (1-2 weeks). However, also this pharmacological treatment is, as known, associated with side effects, such as: inflammation, hemarthrosis, articular infection, crystal arthropathy, articular cartilage atrophy, and steroid-induced arthropathy.

Another strategy for treating osteoarthritis consists in the use of medical devices based on hyaluronic acid, or derivatives thereof, able to restore the viscoelastic nature and natural homeostasis of the synovial fluid. Hyaluronic acid, and cross-linked derivatives thereof to implement its rheological viscosity and viscoelasticity properties, in the form of aqueous formulations directly injected into the joint, have allowed to obtain several benefits in the treatment of osteoarthritis, such as: reduction and inhibition of joint pain, joint lubrication, improvement of arthrosis-related dysfunction, and normalization of joint functions (Kennedy S et al., BC Medical Journal 2010, 52, 404-09; Ayhan and et al., World J Orthop 2014, 5(3), 351 - 61). One of the advantages of the therapy with hyaluronic acid and/or derivatives thereof is the high safety profile that limits the side effects to possible inflammation at the site of injection, although the use of cross-linked hyaluronic acids is associated with a higher incidence of side effects compared to the use of linear hyaluronic acid (Kennedy S et al., BC Medical Journal 2010, 52, 404-09; Onel and et al., Clin Drug Investig 2008, 28, 37-45; Kotevoglu N et al., Rheumatol Int 2006, 26, 325-30). Thanks to all these characteristics, viscosupplementation is today considered an alternative to the pharmacological therapy and, in particular, it is suitable for the treatment of mild forms of osteoarthritis (Kennedy S et al., BC Medical Journal 2010, 52, 404-09). Recent studies have also reported that combined intra-articular administration of hyaluronic acid and non-steroidal anti-inflammatory drugs allows to obtain better benefits than hyaluronic acid alone (Lee SC et al., J Back Musculoskeletal Rehabilitation 2011, 24, 31 -38). The combination of viscosupplementation with pharmacological treatment is therefore receiving increasing attention, especially in view of the possibility of carrying out a treatment that allows to associate the anti-inflammatory and immunosuppressive activity of certain active ingredients, according to the specific degree of severity of osteoarthritis, to the well-known lubricating and visco-elastic properties of formulations based on biopolymers, such as hyaluronic acid and derivatives thereof. In this respect, aqueous compositions based on a cross-linked hyaluronic acid derivative in the presence of a corticosteroid, such as triamcinolone acetonide, where release of the drug from the polymer matrix occurred in a controlled manner, have been described (US2011/0033540). Aqueous formulations of cross-linked hyaluronic acid subsequently added with corticosteroids, such as triamcinolone hexacetonide, have also been reported (US2011/0059918). Similarly, aqueous systems for the release of triamcinolone acetonide from polymeric microparticles have been described, wherein the polymer is not hyaluronic acid, but a copolymer of lactic and glycolic acids (WO2014/153384). In the mentioned systems , the active ingredient is insoluble in water and results homogeneously dispersed thanks to the combination of the polymer matrix and the use of excipients such as PEG, polysorbates and others. The drug release from these systems is determined by the type and amount of excipients, and the degradation of the polymer matrix.

One of the major issues associated with the use of active ingredients in aqueous parenteral formulations is the poor water solubility of the principles *per se.* This issue has been addressed in a number of ways, including the use of solubilizing agents, such as cyclodextrins. Cyclodextrins are widely used as excipients in various pharmaceutical preparations. For example, an injectable aqueous pharmaceutical formulation of diclofenac, polysorbate, and cyclodextrin is described in EP1609481. More generally, water solubilization of other active ingredients, such as triamcinolone, by means of cyclodextrins has been described in several publications (Miro A et al., Carb Polym 2012, 1288-1298; Loftsson T et al., Int J of Pharm 2008, 18-28). It has also been reported that the cyclodextrin-active ingredient association not only has significant effects on the active ingredient, but also on the permeability of the biological membranes to the active ingredient itself (Loftsson T, Pharmazie 2012, 363-70). The combination of cyclodextrins and polysaccharides belonging to the glycosaminoglycan family is described in the literature and, in particular, their association has been shown to be useful to obtain intraarticular formulations for the treatment of osteoarthritis, as reported in WO2015/092516. The use of cyclodextrins in association with pharmaceutical active ingredients and biopolymers, such as hyaluronic acid, to obtain injectable compositions has been described in several reports. WO2013/133647 describes an aqueous composition of hyaluronic acid, cyclodextrin, and piroxicam stabilized by excipients such as PEG and polysorbates; this composition was also tested in an animal model of osteoarthritis, resulting in significant improvements over the use of hyaluronic acid alone (WO2014/200211 ; Park CW et al, Biomol Ther 2014, 22(3), 260-66). A sustained release formulation was reported in which inclusion complexes of cyclodextrin and pyroxicam were dispersed in gels comprising hyaluronic acid and chondroitin sulfate (Choi KH et al., Biomat Res 2008, 13(1),1-6). CN104971039 describes aqueous compositions comprising sulfobutyl ether-beta-cyclodextrin, triamcinolone acetonide acetate and sodium hyaluronate. One of the main advantages of formulations based on hyaluronic acid, cyclodextrin and active ingredient is that the drug is completely or partially solubilized in the matrix, thus limiting the onset of issues related to the presence of precipitates, and/or crystals, such as crystal arthropathy. In addition, the different pharmaceutical form obtained by the addition of cyclodextrin could allow a more efficient use of the active ingredient and, therefore, the amount of drug needed to achieve the therapeutic effect could be reduced, thus limiting the occurrence of other side effects, such as steroid arthropathy and cartilage atrophy. In contrast, one of the major limitations of this strategy is the reduced ability to control drug release from the matrix, in contrast to what is observed in systems composed of cross-linked biopolymers and active ingredient, in the presence or absence of cyclodextrins and other excipients (Quaglia F et al., J Control Rel 2001, 71, 329-37). On the other hand, as previously reported, the use of cross-linked polymer matrixes to obtain viscosupplementation agents, to be used in the treatment of osteoarthritis, is penalized by the higher incidence of side effects. In light of these considerations, it is apparent that the obtainment of a liquid injectable composition composed of linear biopolymers having a homogeneously dispersed active ingredient in it, and whose release is controlled, may lead to improvements in the treatment of osteoarthritis.

The technical problem so far highlighted relates to a complete and uniform solubilization or dispersion of the active ingredient in an aqueous matrix, its stabilization, and its controlled release from the same.

The solution to this problem could be a new type of composition essentially based on the presence of at least two polymers, or two distinct polymer domains that are able to interact with each other in a reversible manner, and without formation of covalent bonds, so to preserve the typical safety profile of linear biopolymers and to provide, at the same time, a dynamic matrix capable of modulating the diffusion, and hence the release, of the active ingredient from the polymer matrix. The addition of cyclodextrin and other excipients/dispersants to the system would finally allow to homogeneously distribute the active ingredient, stabilize its physical form, and ensure and regulate its diffusion from the polymer matrix. EP2021408 describes polysaccharide mixtures composed of polyanions, such as hyaluronic acid, and polycations, such as chitin and chitosan derivatives obtained by a reductive amination reaction with reducing saccharides.

The described compositions are of particular interest since the two polysaccharides which, being polyelectrolytes with different charge, are in principle incompatible with each other in aqueous solution, have been shown to give rise to homogeneously dispersed aqueous solutions, without formation of coacervates characterized by high viscosity and viscoelasticity. Chitosan derivatization, in fact, improves the compatibility of chitosan with polyanionic biopolymers, such as alginic acid and hyaluronic acid, in aqueous solutions.

### Summary

The aim of the present invention is therefore to provide compositions capable of modulating the release of active ingredients from non-crosslinked aqueous polymer matrices for use in the treatment of chronic and acute conditions of musculoskeletal diseases, characterized by inflammatory conditions where it is required to provide a viscosupplementation effect, in addition to the pharmacological effect.

In a first aspect, therefore, an object of the present invention are compositions comprising a clathrate consisting in a cyclodextrin and an active ingredient, wherein the cyclodextrin and active ingredient clathrate is homogeneously dispersed in a polysaccharide polymer matrix in an aqueous solution formed by hyaluronic acid and an oligosaccharide derivative of chitosan with lactose, obtained by a reductive amination reaction of chitosan D-glucosamine, having a degree of substitution of the amine functional group with lactose of at least 40%.

The polysaccharide polymer mixture forms the matrix in which the active ingredient is homogeneously dispersed, thanks to the cyclodextrin contribution, and from which it is released as a function of the polysaccharide composition itself and the type of cyclodextrin.

The compositions object of the invention allow the physical-chemical stabilization of the active ingredient, its complete or partial solubilization in an aqueous environment, when the active ingredient is insoluble in water, and control of its release. Being the polysaccharide polymer matrix in aqueous solution, such compositions are aqueous compositions characterized by viscosity and/or viscoelasticity. Such properties allow a preferential use of these compositions in the treatment of different stages of musculoskeletal diseases wherein the combination of pharmacological and viscosupplementant actions is required.

Therefore, in a second aspect, the compositions object of the invention are for use in the loco-regional treatment of musculoskeletal diseases characterized by inflammatory states, and preferably acute or chronic osteoarticular diseases.

The advantages achievable with the present invention will become more apparent to a person skilled in the art from the following detailed description, and with reference to the following figures.

### Brief Description of the Figures

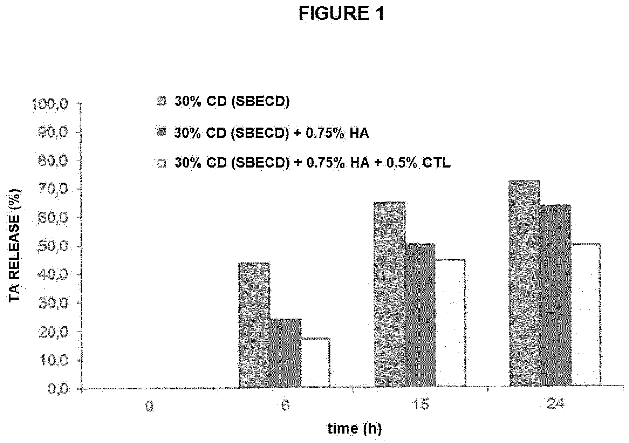

**Figure 1****.** The Figure shows the release kinetics of a 30% (w/v) aqueous solution of cyclodextrin SBECD + triamcinolone acetonide (TA) clathrate, dispersed in a matrix of 0.75% (w/v) aqueous solution of hyaluronic acid (HA), with or without a 0.75% (w/v) aqueous solution of chitlac (CTL).

### Detailed Description of the Invention

The composition for the release of active pharmacological ingredients (briefly identified as API) according to the present invention consists in a polysaccharide polymer matrix, wherein an API and a clathrate formed by a cyclodextrin which includes an active ingredient by complexation is homogeneously dispersed. Such a composition is essentially a hydrogel, since the polymer matrix is composed of an aqueous solution of a polysaccharide mixture, with viscosity and viscoelasticity rheological properties, composed of hyaluronic acid and a chitosan derivative with lactose (hereinafter briefly referred to as chitlac). It may also comprise other excipients and/or dispersants, surfactants, such as polysorbates, polyethylene glycol, and poloxamers.

Unless otherwise specified, in the present invention hyaluronic acid (HA), chitlac (CTL), cyclodextrin (CD), polysorbates, polyethylene glycol, poloxamers, and active pharmacological ingredient are to be intended as follows:
"Hyaluronic acid" means hyaluronic acid and pharmaceutically acceptable salt forms thereof. In other words, herein "hyaluronic acid" refers to hyaluronic acid, pharmaceutically acceptable hyaluronate salts, and mixtures of hyaluronic acid and hyaluronate salts. Hyaluronate salts preferably comprise inorganic salts with alkali cations, such as sodium and potassium. If required, two or more of the above-mentioned compounds may be used. Although in the present invention the molecular weight of hyaluronic acid (hereinafter referred to as MW) is not particularly limited, the range of 500-10,000 KDa, and preferably of 500-2,000 KDa, is recommended wherein MW is determined by intrinsic viscosity measurements and Mark-Hawking equation extrapolation. The term molecular weight as used herein refers to weight average molecular weight. Typically, the measuring method for calculating the weight (average) molecular weight is gel permeation chromatography (GPC method).

Finally, the hyaluronic acid may be obtained from various natural sources, or by recombinant technology fermentation methods.

"Chitlac" means a chitosan derivative suitably functionalized with lactose by substitution of the amine group of chitosan D-glucosamine. The chitosan employable to obtain this derivative can be obtained from several natural sources (e.g. by chitin deacetylation), or by recombinant technology methods, and has an average molecular weight (MW) of up to 1,000 KDa, preferably from 500 to 600 kDa, and more preferably of 200 to 400 kDa wherein MW is determined by gel permeation chromatography. Such chitosan preferably has a deacetylation degree of up to 90%, and the preferred one has a residual acetylation degree of between 10 and 20%. Furthermore, for the purposes of the present invention, the degree of substitution of chitosan D-glucosamine amino groups with lactose is of at least 40%. Preferably, the degree of substitution of chitosan amino groups with such oligosaccharide is comprised in the range from 50% to 70%, and more preferably of 60%.

For the purposes of the present invention, cyclodextrins and their derivatives have the function of incorporating the active ingredient by forming inclusion complexes with it (also called clathrates) and thus acting as a vehicle and means to control its release.

In the present invention, the term "cyclodextrin" means β-cyclodextrin and γ-cyclodextrin ether derivatives. Typically, these ethers or mixtures of ethers include β-cyclodextrin and γ-cyclodextrin, wherein one or more hydroxyl groups are substituted with C₁₋₆-alkyl, hydroxy-C₁₋₆-alkyl, carboxy-C₁₋₆-alkyl, or C₁₋₆-alkyloxycarbonyl groups. Preferably, these compounds include β-cyclodextrin and γ-cyclodextrin, wherein one or more hydroxyl groups are substituted with C₁₋₃-alkyl, hydroxy-C₂₋₄-alkyl, carboxy-C₁₋₂-alkyl groups, and more preferably with methyl, ethyl, hydroxyethyl, hydroxypropyl, hydroxybutyl, carboxymethyl, or carboxymethyl groups. "Cyclodextrins" referred to in the present invention may also be composed of ethers comprising β-cyclodextrin and γ-cyclodextrin, wherein one or more hydroxyl groups are substituted by sulfoalkyl-C₁₋₄-ether groups. In this case both the sulfopropyl ether β-cyclodextrin and the sulfobutylether β-cyclodextrin are appropriate.

The above mentioned "cyclodextrins" have a degree of substitution (DS, degree of substitution of hydroxyl functional groups per unit of glucose) comprised in the range between 0.125 and 3, and more preferably between 0.3 and 2. In addition, one or more hydroxyl groups may be replaced with saccharide groups, such as maltose, glucose, and maltotriose.

Examples of "cyclodextrin" pertaining to the present invention include: 2,6-dimethyl-β-cyclodextrin, 2-hydroxyethyl-β-cyclodextrin, 2-hydroxypropyl-β-cyclodextrin, (2-carboxymethoxy)propyl-β-cyclodextrin 2-hydroxypropyl-γ-cyclodextrin, sulfobutylether (7)-β-cyclodextrin, and the preferred among these are sulfobutylether (7)-β-cyclodextrin (hereinafter referred to as SBECD), 2-hydroxypropyl-β-cyclodextrin (hereinafter referred to as HPβCD), and 2-hydroxypropyl-γ-cyclodextrin (hereinafter referred to as HPγCD).

"Polysorbates" means essentially commercial products such as Polysorbate 20, Polysorbate 60, and Polysorbate 80.

In the present invention, "polyethylene glycol" means a polyethylene glycol with an average molecular weight comprised in the range from 200 to 100,000 Da, and whose structure contemplates the presence of a hydroxyl terminal group, an initiator group selected, for example, from amines, carboxy and hydroxyl groups. Any molecular weight can be used, the preferred one has an average molecular weight in the range from 400 to 10,000. Both linear and branched polymers can be used. In the present invention, poloxamers means copolymer of polyoxyethylene-polyoxypropylene, or block polymers commonly known under the trade name Pluronic^{®} F-68, Pluronic^{®} F-127 or Poloxamer, Poloxamer 188.

The "pharmacological active ingredients" (hereinafter referred to as APIs) pertaining to the present invention are selected, irrespectively of their solubility or insolubility in aqueous solutions, from anti-infectives such as, for example, antibiotics, anti-arthritis drugs, corticosteroids and non-steroidal anti-inflammatory agents. Examples of active ingredient relevant to the present invention selected from corticosteroids are triamcinolone acetonide and triamcinolone hexacetonide, and from non-steroidal anti-inflammatory agents are pharmaceutically acceptable forms of diclofenac, piroxicam, ketorolac and ibuprofen.

Advantageously, for the purposes of the present invention, the release control depends on the specific combination of cyclodextrin, hyaluronic acid, and chitlac. Infact, while it is known that the diffusion of species in solution is affected by the medium viscosity - therefore hyaluronic acid and chitlac amounts and ratio- we found out that it is also possible to introduce further degree of control on the basis of the interaction of the polymeric matrix with the clathrate, in such a way that the same API in different cyclodextrin clathrates will be released in a different manner from the same Chitlac/HYAC combination.

The polysaccharide matrix composed of hyaluronic acid and chitlac is comprised between 0.5% and 4%, and the individual polysaccharide components are comprised between 0.25% and 2%, respectively. The ratios of hyaluronic acid to chitlac are comprised between 1:3 and 10:1, and more preferably between 1:1 and 5:1.

The cyclodextrin is comprised between 1% and 30%. The specific amount depends on the actual amount of active pharmacological ingredient to be solubilized and, in general, the ratio of cyclodextrin to active ingredient is within the range of 3-100.

The active ingredient, homogeneously dispersed in the formulation, is present in amounts comprised between 0.05% and 2.50%, by weight. The specific amount depends on the type of active ingredient and its therapeutic dosage.

Finally, polysorbates, poloxamers, and propylene glycol may be used to stabilize the composition, without precluding drug release control, which is determined by the polymer matrix and cyclodextrin. Typically, their amount, by weight, is comprised between 0.02% and 0.10% in the case of polysorbates and poloxamers, whereas the amount is between 0.5% and 10% in the case of polyethylene glycol.

The present invention further provides a method for preparing the new matrix for active ingredients controlled release using non-crosslinked polysaccharides and cyclodextrins. Briefly, the cyclodextrin, the active ingredient and optional excipients are mixed in an aqueous solvent, and the system is stirred for a time sufficient to obtain the solubilization of the active ingredient, according to the amount of cyclodextrin and excipient employed. Then, an aqueous solution of chitlac is added and, under stirring, hyaluronic acid is added as a solid. The resulting formulation is stirred until a homogeneous preparation is obtained. The solvent commonly used for the present invention is a saline solution or phosphate buffered saline solution.

The use of the non-crosslinked polysaccharide matrix, in the presence of cyclodextrin and excipients, for the release of active ingredients described in the present invention allows to obtain injectable viscoelastic compositions containing active ingredients, whose release can be modulated according to the relative amounts of polymers and cyclodextrins type, without using cross-linked polymer matrices, thanks to an unprecedented tuning of supramolecular interactions between cyclodextrin API clathrates and the polyelectrolytes matrix based on the specific combination of cyclodextrin and API sizes and charges. The charge and size of clathrates play an important role in the diffusion through the polyelectrolyte matrix. Surprisingly, we have found that different clathrates obtained from different cyclodextrin with the same API are characterized by different release rates, even in absence of polymer matrix, and that the addition of the polyelectrolyte matrix allows a further tuning of the release kinetic which is related to the specific interactions between clathrate and polymer matrix.

The present invention is hereinafter described in detail with reference to specific examples in order to illustrate the principles of the invention. However, this should not be interpreted as limiting the scope of the present invention. The following examples are intended to fully explain the invention as will be apparent to someone skilled in the art.

As will be apparent from the examples below, variations in the solubility of the active ingredient were found by combining the cyclodextrin and hyaluronic acid formulation. The same can be observed for the cyclodextrin-active ingredient system, added with either chitlac or chitlac and hyaluronic acid.

The solubility variations found may be attributed to different effects of the polymer matrix on the cyclodextrin-active ingredient clathrate. Differences in solubilizing power, at the same concentration and type of cyclodextrin and active ingredient, were found to be dependent on the type of polysaccharide employed, the co-presence of other polysaccharides, and the specific ratio between the two polysaccharides. Without wishing to be bound by any theory, a possible explanation might be that different polysaccharide ratios may generate solutions containing soluble polyelectrolyte of different characteristics. It is possible to speculate that these different supramolecular macrostructures are able to stabilize the cyclodextrin-active ingredient clathrates differently, and thus, ultimately, allow different solubilization degrees which therefore impact the stabilization and diffusion of the clathrates through the matrix. The effect of polymeric components on the solubilizing capacity of cyclodextrins has been discussed in the literature (Loftsson et al, Journal of Pharmaceutical Sciences 2012, 101, 3019-32), however, there are no indications regarding a synergistic effect of polycation and polyanion polysaccharide components on the solubility and diffusion through the matrix of cyclodextrin-drug inclusion complexes.

The addition of other polymers or surfactants as excipients, such as polysorbate and/or PEG, does not seem to significantly affect the dispersion/solubilization of the CD+API inclusion complex and, therefore, there is no preclusion from using them in association with a polysaccharide matrix.

However, it is to be noted that the active ingredient release is increasingly rapid when the system is deprived of the polysaccharide matrix, while the introduction of a single polymer component, such as hyaluronic acid, in the system allows to slow down the release rate. Moreover, when both polymers are present in the system, a further slowdown of the active ingredient release is observed and surprisingly switching from one cyclodextrin to another allows to design faster or slower release systems.

### EXAMPLES

The preparation of compositions for controlled release of active pharmaceutical ingredient according to the invention, wherein a cyclodextrin and API clathrate is dispersed in a polyanion and polycation polysaccharide matrix, was performed by studying every time the effects of the of the composition individual components.

The experiments described in the examples below were carried out using the following polysaccharides
Hyaluronic Acid (HA): 1-1.6 MDa (1000-1600 kDa) of pharmaceutical grade suitable for human administration, obtained by biofermentation.

Chitlac Hydrochloride (CTL): chitlac hydrochloride was obtained by addition of aqueous hydrochloric acid to a chitlac solution in water until pH 2.5 was reached. Then, the polymer salt was precipitated with methanol, filtered on a sintered glass filter (gooch), and the collected solid washed with methanol (3x) and dried. The chitlac used for salt preparation is characterized by a degree of lactose replacement comprised between 50 and 70%, and was obtained from a 200-400 kDa chitosan with a residual acetylation degree of approximately 15%.

Polysaccharide stock solutions of known concentration were prepared using water for injectable solutions as described below.

Chitlac 2% in PBS 1X: 1.6g of chitlac hydrochloride were dissolved in 66.08 mL of water for injectable solutions, and 5.92 mL of 0.5 M NaOH were then added dropwise to the solution obtained. The solution was then added with 8 mL of 10 X phosphate buffered saline (PBS 10 X: 1370 mM NaCl, 27 mM KCI, 81 mM Na₂HPO₄, 17.6 mM NaH₂PO₄) and stirred for a further 15 minutes.

**Example 1.** Aqueous composition of triamcinolone acetonide (TA, 0.44%) included in sulfobutylether-7-beta-cyclodextrin (SBECD, 30%) in a matrix of hyaluronic acid (HA, 0.25%) and chitlac (CTL, 0.75%).

1.5 g of sulfobutylether-7-beta-cyclodextrin were dissolved in 3.125 mL of phosphate buffered saline solution (PBS 1X: 137 mM NaCl, 2.7 mM KCI, 8.1 mM Na₂HPO₄, 1.76 mM NaH₂PO₄), 22 mg of triamcinolone acetonide were subsequently added, and the system thus obtained was stirred for 16 h at room temperature. 1.875 mL of a 2% (w/v) solution of chitlac in phosphate buffered saline solution (PBS 1X: 137 mM NaCl, 2.7 mM KCI, 8.1 mM Na₂HPO₄, 1.76 mM NaH₂PO₄) were then added, the system stirred for 15 minutes, and then added with 37.5 mg of hyaluronic acid. The mixture thus obtained was stirred at 60°C for 2 h, and at room temperature for 16 h. The pH of the solution was 7.4.

| **#** | **SBECD (g, % w/v)** | **TA (g, % w/v)** | **HA (g, % w/v)** | **CTL (g, % w/v)** |
|---|---|---|---|---|
| **1** | 1.5 (30% w/v) | 0.022 (0.44% w/v) | 0.0125 (0.25% w/v) | 0.0375 (0.75% w/v) |

**Examples 2-8.** Aqueous compositions of triamcinolone acetonide (TA, 0.44 and 0.27%) included in sulfobutylether-7-beta-cyclodextrin (SBECD, 15 and 30%) in a matrix of hyaluronic acid (HA) and chitlac (CTL) at varying concentrations.

The formulations of Examples 2-8 were obtained following the procedure illustrated in Example 1, and using the amounts shown in the table below. The pH of the solutions was 7.4.

| **#** | **SBECD (g, % w/v)** | **TA (g, % w/v)** | **V (mL) PBS 1X** | **V (mL) CTL 2% (w/v) in PBS 1X** | **HA (g, % w/v)** | **CTL (g, % w/v)** |
|---|---|---|---|---|---|---|
| **2** | 1.5 (30%) | 0.022 (0.44%) | 3.125 | 1.875 | 0.0375 (0.75%) | 0.0375 (0.75%) |
| **3** | 1.5 (30%) | 0.022 (0.44%) | 3.125 | 1.875 | 0.050 (1.0%) | 0.0375 (0.75) |
| **4** | 1.5 (30%) | 0.022 (0.44%) | 3.75 | 1.25 | 0.0375 (0.75%) | 0.025 (0.50%) |
| **5** | 1.5 (30%) | 0.022 (0.44%) | 4.375 | 0.625 | 0.050 (1%) | 0.0125 (0.25%) |
| **6** | 1.5 (30%) | 0.022 (0.44%) | 4.725 | 0.275 | 0.057 (1.14%) | 0.0055 (0.11%) |
| **7** | 1.5 (30%) | 0.022 (0.44%) | 3.00 | 2.00 | 0.060 (1.20%) | 0.040 (0.80%) |
| **8** | 0.75 (15%) | 0.0135 (0.27%) | 3.75 | 1.25 | 0.0375 (0.75%) | 0.025 (0.50%) |

**Example 9.** Aqueous composition of triamcinolone acetonide (TA, 1.2%) included in sulfobutylether-7-beta-cyclodextrin (SBECD, 30%) and polysorbate (0.05%) in a matrix of hyaluronic acid (HA, 0.75%) and chitlac (CTL, 0.50%).

1.5 g of sulfobutylether-7-beta-cyclodextrin were dissolved in 3.75 mL of phosphate buffered saline solution (PBS 1X: 137 mM NaCl, 2.7 mM KCI, 8.1 mM Na₂HPO₄, 1.76 mM NaH₂PO₄), 2.5 mg of polysorbate 20 and 60 mg of triamcinolone acetonide were subsequently added, and the system thus obtained was stirred for 16 h at room temperature. 1.25 mL of a 2% (w/v) solution of chitlac in phosphate buffered saline solution (PBS 1X: 137 mM NaCl, 2.7 mM KCI, 8.1 mM Na₂HPO₄, 1.76 mM NaH₂PO₄) were then added, the system stirred for 15 minutes, and then added with 37.5 mg of hyaluronic acid. The mixture thus obtained was stirred at 60°C for 2 h, and at room temperature for 16 h. The pH of the solution was 7.4.

| **#** | **SBECD (g, % w/v)** | **TA (g, % w/v)** | **POLYSORBATE 20 (mg, % w/v)** | **HA (g, % w/v)** | **CTL (g, % w/v)** |
|---|---|---|---|---|---|
| **9** | 1.5 (30%) | 0.060 (1.2%) | 2.5 (0.05%) | 0.0375 (0.75%) | 0.025 (0.50%) |

**Examples 10-11.** Aqueous compositions of triamcinolone acetonide (TA, 1.2%) included in sulfobutylether-7-beta-cyclodextrin (SBECD, 15%) and polysorbate (0.05%) in a matrix of hyaluronic acid (HA) and chitlac (CTL) at varying concentrations.

The formulations of Examples 10-11 were obtained following the procedure illustrated in Example 9, and using the amounts shown in the table below. The pH of the solutions was 7.4.

| **#** | **SBECD (g, % w/v)** | **TA (g)** | **POLYSORBATE 20 (mg, % w/v)** | **V (mL) PBS 1X** | **V (mL) CTL 2% (w/v) in PBS 1X** | **HA (g, % w/v)** | **CTL (g, % w/v)** |
|---|---|---|---|---|---|---|---|
| **10** | 0.75 (15%) | 0.060 (1.2%) | 2.5 (0.05%) | 3.75 | 1.25 | 0.0375 (0.75%) | 0.025 (0.50%) |
| **11** | 0.75 (15%) | 0.060 (1.2%) | 2.5 (0.05%) | 3.125 | 1.875 | 0.060 (1.25%) | 0.0375 (0.75%) |

**Example 12.** Aqueous composition of triamcinolone acetonide (TA, 1.2%) included in sulfobutylether-7-beta-cyclodextrin (SBECD, 15%), polysorbate (0.05%) and PEG 5000 (9%) in a matrix of hyaluronic acid (HA, 0.75%) and chitlac (CTL, 0,50%). 0.75 g of sulfobutylether-7-beta-cyclodextrin were dissolved in 3.125 mL of phosphate buffered saline solution (PBS 1X: 137 mM NaCl, 2.7 mM KCI, 8.1 mM Na₂HPO₄, 1.76 mM NaH₂PO₄), 2.5 mg of polysorbate 20 and 0.45 g of polyethylene glycol (PEG 5000), and 60 mg of triamcinolone acetonide were subsequently added, and the system thus obtained was stirred for 16 h at room temperature. 1.875 mL of a 2% (w/v) solution of chitlac in phosphate buffered saline solution (PBS 1X: 137 mM NaCl, 2.7 mM KCI, 8.1 mM Na₂HPO₄, 1.76 mM NaH₂PO₄) were then added, the system stirred for 15 minutes, and then added with 60 mg of hyaluronic acid. The mixture thus obtained was stirred at 60°C for 2 h, and at room temperature for 16 h. The pH of the solutions was 7.4.

| **#** | **SBECD (g, % w/v)** | **TA (g, % w/v)** | **POLYSORBATE 20 (mg, % w/v)** | **PEG 5000 (g, % w/v)** | **HA (g, % w/v)** | **CTL (g, % w/v)** |
|---|---|---|---|---|---|---|
| **12** | 0.75 (15%) | 0.060 (1.2%) | 2.5 (0.05%) | 0.450 (9%) | 0.0375 (0.75%) | 0.025 (0.50% w/v) |

**Examples 13-20.** Aqueous compositions of triamcinolone acetonide (TA, 0.44%) included in hydroxypropyl-β-cyclodextrin (HPβCD, 30%) in a matrix of hyaluronic acid (HA) and chitlac (CTL) at varying concentrations.

The formulations of Examples 13-20 were obtained following the procedure reported in Example 1 using hydroxypropyl-β-cyclodextrin, and the amounts shown in the table below. The pH of the solutions was 7.4.

| **#** | **HPβCD (g, % w/v)** | **TA (g, % w/v)** | **V (mL) PBS 1X** | **V (mL) CTL 2% (w/v) in PBS 1X** | **HA (g, % w/v)** | **CTL (g, % w/v)** |
|---|---|---|---|---|---|---|
| **13** | 1.5 (30%) | 0.022 (0.44%) | 3.125 | 1.875 | 0.0125 (0.25%) | 0.0375 (0.75%) |
| **14** | 1.5 (30%) | 0.022 (0.44%) | 3.125 | 1.875 | 0.0375 (0.75%) | 0.0375 (0.75%) |
| **15** | 1.5 (30%) | 0.022 (0.44%) | 3.125 | 1.875 | 0.050 (1.0%) | 0.0375 (0.75%) |
| **16** | 1.5 (30%) | 0.022 (0.44%) | 3.75 | 1.25 | 0.0375 (0.75%) | 0.025 (0.50%) |
| **17** | 1.5 (30%) | 0.022 (0.44%) | 4.375 | 0.625 | 0.050 (1%) | 0.0125 (0.25%) |
| **18** | 1.5 (30%) | 0.022 (0.44%) | 4.725 | 0.275 | 0.057 (1.14%) | 0.0055 (0.11%) |
| **19** | 1.5 (30%) | 0.022 (0.44%) | 3.00 | 2.00 | 0.060 (1.20%) | 0.040 (0.80%) |
| **20** | 0.75 (15%) | 0.010 (0.20%) | 3.75 | 1.25 | 0.0375 (0.75%) | 0.025 (0.50%) |

**Examples 21-23.** Aqueous compositions of triamcinolone acetonide (TA, 1.2%) included in hydroxypropyl-β-cyclodextrin (HPβCD) and polysorbate (0.05%) in a matrix of hyaluronic acid (HA) and chitlac (CTL) at varying concentrations.

The formulations of Examples 21-23 were obtained following the procedure reported in Example 9, and using the amounts shown in the table below. The pH of the solutions was 7.4.

| **#** | **HPβCD (g, % w/v)** | **TA (g, % w/v)** | **POLYSORBATE 20 (mg, % w/v)** | **V (mL) PBS 1X** | **V (mL) CTL 2% (w/v) in PBS 1X** | **HA (g, % w/v)** | **CTL (g, % w/v)** |
|---|---|---|---|---|---|---|---|
| **21** | 1.50 30%) | 0.060 (1.2%) | 2.5 (0.05%) | 3.75 | 1.25 | 0.0375 (0.75%) | 0.025 (0.50%) |
| **22** | 0.75 (15%) | 0.060 (1.2%) | 2.5 (0.05%) | 3.75 | 1.25 | 0.0375 (0.75%) | 0.025 (0.50%) |
| **23** | 0.75 (15%) | 0.060 (1.2%) | 2.5 (0.05%) | 3.125 | 1.875 | 0.060 (1.25%) | 0.0375 (0.75%) |

**Example 24.** Aqueous composition of triamcinolone acetonide (TA, 1.2%) included in hydroxypropyl-β-cyclodextrin (HPβCD 15%), polysorbate (0.05%) and PEG 5000 (9%) in a matrix of hyaluronic acid (HA, 1,25%) and chitlac (CTL, 0.75%) at varying concentrations.

The formulation of Example 24 was obtained following the procedure reported in Example 12, and using the amounts shown in the table below. The pH of the solution was 7.4.

| **#** | **HPβCD (g)** | **TA (g)** | **POLYSORBATE 20 (mg)** | **PEG 5000 (g)** | **V (mL) PBS 1X** | **V (mL) CTL 2% (w/v) in PBS 1X** | **HA (g)** | **CTL (g)** |
|---|---|---|---|---|---|---|---|---|
| **24** | 0.75 (15%) | 0.060 (1.2%) | 2.5 (0.05%) | 0.450 (9%) | 3.125 | 1.875 | 0.060 (1.25%) | 0.0375 (0.75%) |

**Examples 25-32.** Aqueous compositions of triamcinolone hexacetonide (THA, 0.60 and 0.16%) included in sulfobutylether-7-beta-cyclodextrin (SBECD, 15 and 30%) in a matrix of hyaluronic acid (HA) and chitlac (CTL) at varying concentrations.

The formulations of Examples 25-32 were obtained following the procedure reported in Example 1, and using the amounts shown in the table below. The pH of the solutions was 7.4.

| **#** | **SBECD (g, % w/v)** | **THA (g, % w/v)** | **V (mL) PBS 1X** | **V (mL) CTL 2% (w/v) in PBS 1X** | **HA (g, % w/v)** | **CTL (g, % w/v)** |
|---|---|---|---|---|---|---|
| **25** | 1.5 (30%) | 0.030 (0.60%) | 3.125 | 1.875 | 0.0125 (0.25%) | 0.0375 (0.75%) |
| **26** | 1.5 (30%) | 0.030 (0.60%) | 3.125 | 1.875 | 0.0375 (0.75%) | 0.0375 (0.75%) |
| **27** | 1.5 (30%) | 0.030 (0.60%) | 3.125 | 1.875 | 0.050 (1.0%) | 0.0375 (0.75%) |
| **28** | 1.5 (30%) | 0.030 (0.60%) | 3.75 | 1.25 | 0.0375 (0.75%) | 0.025 (0.50%) |
| **29** | 1.5 (30%) | 0.030 (0.60%) | 4.375 | 0.625 | 0.050 (1%) | 0.0125 (0.25%) |
| **30** | 1.5 (30%) | 0.030 (0.60%) | 4.725 | 0.275 | 0.057 (1.14%) | 0.0055 (0.11%) |
| **31** | 1.5 (30%) | 0.030 (0.60%) | 3.00 | 2.00 | 0.060 (1.20%) | 0.040 (0.80%) |
| **32** | 0.75 (15%) | 0.008 (0.16%) | 3.75 | 1.25 | 0.0375 (0.75%) | 0.025 (0.50%) |

**Examples 33-36.** Aqueous compositions of triamcinolone hexacetonide (THA, 0.60 and 1.2%) included in sulfobutylether-7-beta-cyclodextrin (SBECD, 15 and 30%) and polysorbate (0.05%) in a matrix of hyaluronic acid (HA) and chitlac (CTL) at varying concentrations.

The formulations of Examples 33-36 were obtained following the procedure reported in Example 9, and using the amounts shown in the table below. The pH of the solutions was 7.4.

| **#** | **SBECD (g, % w/v)** | **THA (g, % w/v)** | **POLYSORBATE 20 (mg, % w/v)** | **V (mL) PBS 1X** | **V (mL) CTL 2% (w/v) in PBS1X** | **HA (g, % w/v)** | **CTL (g, % w/v)** |
|---|---|---|---|---|---|---|---|
| 33 | 1.5 (30%) | 0.030 (0.60%) | 2.5 (0.05%) | 3.75 | 1.25 | 0.0375 (0.75%) | 0.025 (0.50%) |
| **34** | 1.50 (30%) | 0.030 (0.60%) | 2.5 (0.05%) | 3.75 | 1.25 | 0.0375 (0.75%) | 0.025 (0.50%) |
| **35** | 0.75 (15%) | 0.060 (1.2%) | 2.5 (0.05%) | 3.75 | 1.25 | 0.0375 (0.75%) | 0.025 (0.50%) |
| **36** | 0.75 (15%) | 0.060 (1.2%) | 2.5 (0.05%) | 3.125 | 1.875 | 0.060 (1.25%) | 0.0375 (0.75%) |

**Example 37.** Aqueous composition of triamcinolone hexacetonide (THA, 1.2%) included in sulfobutylether-7-beta-cyclodextrin (SBECD, 15%), polysorbate (0.05%) and PEG 5000 (9%) in a matrix of hyaluronic acid (HA, 1.25%) and chitlac (CTL, 0.75%).

The formulation of Examples 37 was obtained following the procedure reported in Example 12, and using the amounts shown in the table below. The pH of the solution was 7.4.

| **#** | **SBECD (g, % w/v)** | **THA (g, % w/v)** | **POLYSORBATE 20 (mg, % w/v)** | **PEG 5000 (g, % w/v)** | **V (mL) PBS 1X** | **V (mL) CTL 2% (w/v) in PBS1X** | **HA (g, % w/v)** | **CTL (g, % w/v)** |
|---|---|---|---|---|---|---|---|---|
| **37** | 0.75 (15%) | 0.060 (1.2%) | 2.5 (0.05%) | 0.450 (9%) | 3.125 | 1.875 | 0.060 (1.25%) | 0.0375 (0.75%) |

**Examples 38-45.** Aqueous compositions of triamcinolone hexacetonide (THA, 0.70 and 0.17%) included in hydroxypropyl-β-cyclodextrin (HPβCD, 15 and 30%) in a matrix of hyaluronic acid (HA) and chitlac (CTL) at varying concentrations.

The formulations of Examples 38-45 were obtained following the procedure reported in Example 1, and using the amounts shown in the table below. The pH of the solutions was 7.4.

| **#** | **HPβCD (g, % w/v)** | **THA (g, % w/v)** | **V (mL) PBS 1X** | **V (mL) CTL 2% (w/v) in PBS 1X** | **HA (g, % w/v)** | **CTL (g, % w/v)** |
|---|---|---|---|---|---|---|
| **38** | 1.5 (30%) | 0.035 (0.70%) | 3.125 | 1.875 | 0.0125 (0.25%) | 0.0375 (0.75%) |
| **39** | 1.5 (30%) | 0.035 (0.70%) | 3.125 | 1.875 | 0.0375 (0.75%) | 0.0375 (0.75%) |
| **40** | 1.5 (30%) | 0.035 (0.70%) | 3.125 | 1.875 | 0.050 (1.0%) | 0.0375 (0.75%) |
| **41** | 1.5 (30%) | 0.035 (0.70%) | 3.75 | 1.25 | 0.0375 (0.75%) | 0.025 (0.50%) |
| **42** | 1.5 (30%) | 0.035 (0.70%) | 4.375 | 0.625 | 0.050 (1%) | 0.0125 (0.25%) |
| **43** | 1.5 g (30%) | 0.035 (0.70%) | 4.725 | 0.275 | 0.057 (1.14%) | 0.0055 (0.11%) |
| **44** | 1.5 (30%) | 0.035 (0.70%) | 3.00 | 2.00 | 0.060 (1.20%) | 0.040 (0.80%) |
| **45** | 0.75 (15%) | 0.0085 (0.17%) | 3.75 | 1.25 | 0.0375 (0.75%) | 0.025 (0.50%) |

**Examples 46-48.** Aqueous compositions of triamcinolone hexacetonide (THA, 1.2%) included in hydroxypropyl-β-cyclodextrin (HPβCD, 15 and 30%) and polysorbate (0.05%) in a matrix of hyaluronic acid (HA) and chitlac (CTL) at varying concentrations.

The formulations of Examples 46-48 were obtained following the same procedure reported in Example 9, and using the amounts shown in the table below. The pH of the solutions was 7.4.

| **#** | **HPβCD (g, % w/v)** | **THA (g, % w/v)** | **POLYSORBATE 20 (mg, % w/v)** | **V (mL) PBS 1X** | **V (mL) CTL 2% (w/v) in PBS 1X** | **HA (g, % w/v)** | **CTL (g, % w/v)** |
|---|---|---|---|---|---|---|---|
| **46** | 1.50 (30%) | 0.060 (1.2%) | 2.5 (0.05%) | 3.75 | 1.25 | 0.0375 (0.75%) | 0.025 (0.50%) |
| **47** | 0.75 (15%) | 0.060 (1.2%) | 2.5 (0.05%) | 3.75 | 1.25 | 0.0375 (0.75%) | 0.025 (0.50%) |
| **48** | 0.75 (15% w/v) | 0.060 (1.2%) | 2.5 (0.05%) | 3.125 | 1.875 | 0.060 (1.25%) | 0.0375 (0.75%) |

**Example 49.** Aqueous composition of triamcinolone hexacetonide (THA, 1.2%) included in hydroxypropyl-β-cyclodextrin (HPβCD, 15%), polysorbate (0.05%) and PEG 5000 (9%) in a matrix of hyaluronic acid (HA, 1.25%) and chitlac (CTL, 0.75%). The formulation of Examples 49 was obtained following the procedure reported in Example 12, and using the amounts shown in the table below. The pH of the solutions was 7.4.

| **#** | **HPβCD (g, % w/v)** | **THA (g, % w/v)** | **POLYSORBATE 20 (mg, % w/v)** | **PEG 5000 (g, % w/v)** | **V (mL) PBS 1X** | **V (mL) CTL 2% (w/v) in PBS 1X** | **HA (g, % w/v)** | **CTL (g, % w/v)** |
|---|---|---|---|---|---|---|---|---|
| **49** | 0.75 (15%) | 0.060 (1.2%) | 2.5 (0.05%) | 0.450 (9%) | 3.125 | 1.875 | 0.060 (1.25%) | 0.0375 (0.75%) |

**Examples 50-51.** Aqueous compositions of diclofenac sodium salt (DCNa, 1.5%) included in sulfobutylether-7-beta-cyclodextrin (SBECD, 5%) in a matrix of hyaluronic acid (HA) and chitlac (CTL) at varying concentrations.

The formulations of Examples 50-51 were obtained following the same procedure reported in Example 1, and using the amounts shown in the table below. The pH of the solutions was 7.4.

| **#** | **SBECD (g, % w/v)** | **DCNa (g, % w/v)** | **V (mL) PBS 1X** | **V (mL) CTL 2% (w/v) in PBS 1X** | **HA (g, % w/v)** | **CTL (g, % w/v)** |
|---|---|---|---|---|---|---|
| **50** | 0.25 (5%) | 0.075 (1.5%) | 3.75 | 1.25 | 0.0375 (0.75%) | 0.025 (0.25%) |
| **51** | 0.25 (5%) | 0.075 (1.5%) | 3.125 | 1.875 | 0.0625 (1.25%) | 0.0375 (0.75%) |

**Example 52.** Aqueous composition of diclofenac sodium salt (DCNa, 1.5%) included in sulfobutylether-7-beta-cyclodextrin (SBECD, 5%) and polysorbate (0.05%) in a matrix of hyaluronic acid (HA, 1.25%) and chitlac (CTL, 0.75%).

The formulation of Example 52 was obtained following the same procedure reported in Example 9, and using the amounts shown in the table below. The pH of the solution was 7.4.

| **#** | **SBECD (g, % w/v)** | **DCNa (g, % w/v)** | **POLYSORBATE 20 (mg, % w/v)** | **V (mL) PBS 1X** | **V (mL) CTL 2% (w/v) in PBS 1X** | **HA (g, % w/v)** | **CTL (g, % w/v)** |
|---|---|---|---|---|---|---|---|
| **52** | 0.25 (5%) | 0.075 (1.5% ) | 2.5 (0.05% w/v) | 3.125 | 1.875 | 0.0625 (1.25%) | 0.0375 (0.75%) |

**Example 53.** Aqueous composition of diclofenac sodium salt (DCNa, 1.5%) included in hydroxypropyl-beta-cyclodextrin (HPβCD, 5%) in a matrix of hyaluronic acid (HA, 0.75%) and chitlac (CTL, 0.50%).

The formulation of Example 53 was obtained following the same procedure reported in Example 1, and using the amounts shown in the table below. The pH of the solutions was 7.4.

| **#** | **HPβCD (g, % w/v)** | **DCNa (g, % w/v)** | **V (mL) PBS 1X** | **V (mL) CTL 2% (w/v) in PBS 1X** | **HA (g, % w/v)** | **CTL (g, % w/v)** |
|---|---|---|---|---|---|---|
| **53** | 0.25 (5%) | 0.075 (1.5%) | 3.75 | 1.25 | 0.0375 (0.75%) | 0.025 (0.5%) |

**Example 54.** Aqueous composition of diclofenac sodium salt (DCNa, 1.5%) included in hydroxypropyl-beta-cyclodextrin (HPβCD, 5%) in a matrix of hyaluronic acid (HA, 1.25%) and chitlac (CTL, 0.75%).

The formulation of Example 54 was obtained following the same procedure reported in Example 1, and using the amounts shown in the table below. The pH of the solutions was 7.4.

| **#** | **HPβCD (g, % w/v)** | **DCNa (g, % w/v)** | **V (mL) PBS 1X** | **V (mL) CTL 2% (w/v) in PBS 1X** | **HA (g, % w/v)** | **CTL (g, % w/v)** |
|---|---|---|---|---|---|---|
| **54** | 0.25 (5%) | 0.075 (1.5%) | 3.125 | 1.875 | 0.0625 (1.25%) | 0.0375 (0.75%) |

**Example 55.** Aqueous composition of diclofenac sodium salt (DCNa, 1.5%) included in hydroxypropyl-beta-cyclodextrin (HPβCD, 5%) and polysorbate (0.05%) in a matrix of hyaluronic acid (HA, 1.25%) and chitlac (CTL, 0.75%).

The formulation of Example 55 was obtained following the same procedure reported in Example 9, and using the amounts shown in the table below. The pH of the solution was 7.4.

| **#** | **HPβCD (g, % w/v)** | **DCNa (g, % w/v)** | **POLYSORBATE 20 (mg, % w/v)** | **V (mL) PBS 1X** | **V (mL) CTL 2% (w/v) in PBS 1X** | **HA (g, % w/v)** | **CTL (g, % w/v)** |
|---|---|---|---|---|---|---|---|
| **55** | 0.25 (5%) | 0.075 (1.5%) | 2.5 (0.05%) | 3.125 | 1.875 | 0.0625 (1.25%) | 0.0375 (0.75%) |

**Example 56.** Aqueous composition of diclofenac sodium salt (DCNa, 1.5%) included in hydroxypropyl-gamma-cyclodextrin (HPγCD, 5%) in a matrix of hyaluronic acid (HA, 0.75%) and chitlac (CTL, 0.50%).

The formulation of Example 56 was obtained following the same procedure reported in Example 1, and using the amounts shown in the table below. The pH of the solution was 7.4.

| **#** | **HPγCD (g, % w/v)** | **DCNa (g, % w/v)** | **V (mL) PBS 1X** | **V (mL) CTL 2% (w/v) in PBS 1X** | **HA (g, % w/v)** | **CTL (g, % w/v)** |
|---|---|---|---|---|---|---|
| **56** | 0.25 (5%) | 0.075 (1.5%) | 3.75 | 1.25 | 0.0375 (0.75%) | 0.025 (0.5%) |

**Example 57.** Comparative example of aqueous composition of triamcinolone acetonide (TA, 0.44%) included in sulfobutylether-7-beta-cyclodextrin (SBECD, 30%) in a chitlac matrix (CTL, 0.75%).

1.5 g of sulfobutylether-7-beta-cyclodextrin were dissolved in 3.125 mL of phosphate buffered saline solution (PBS 1X: 137 mM NaCl, 2.7 mM KCI, 8.1 mM Na₂HPO₄, 1.76 mM NaH₂PO₄), 22 mg of triamcinolone acetonide were subsequently added, and the system thus obtained was stirred for 16 h at room temperature. 1.875 mL of a 2% (w/v) solution of chitlac in phosphate buffered saline solution (PBS 1X: 137 mM NaCl, 2.7 mM KCI, 8.1 mM Na₂HPO₄, 1.76 mM NaH₂PO₄) were then added, and the system stirred for further 30 minutes. The pH of the solution was 7.4.

| **#** | **SBECD (g, % w/v)** | **TA (g, % w/v)** | **CTL (g, % w/v)** |
|---|---|---|---|
| **57** | 1.5 (30% w/v) | 0.022 (0.44% w/v) | 0.0375 (0.75% w/v) |

**Example 58.** Comparative example of aqueous composition of triamcinolone acetonide (TA, 0.44%) included in hydroxypropyl-beta-cyclodextrin (HPβCD, 30%) in a chitlac matrix (CTL, 0.75%).

The formulation of Example 58 was obtained following the same procedure reported in Example 57, and using the amounts shown in the table below. The pH of the solution was 7.4.

| **#** | **HPβCD (g, % w/v)** | **TA (g, % w/v)** | **CTL (g, % w/v)** |
|---|---|---|---|
| **58** | 1.5 (30% w/v) | 0.022 (0.44% w/v) | 0.0375 (0.75% w/v) |

**Example 59.** Comparative example of aqueous composition of triamcinolone hexacetonide (THA, 0.7%) included in sulfobutylether-7-beta-cyclodextrin (SBECD, 30%) in a chitlac matrix (CTL, 0.75%).

The formulation of Example 59 was obtained following the same procedure reported in Example 57, and using the amounts shown in the table below. The pH of the solution was 7.4.

| **#** | **SBECD (g, % w/v)** | **THA (g, % w/v)** | **CTL (g, % w/v)** |
|---|---|---|---|
| **58** | 1.5 (30% w/v) | 0.030 (0.6% w/v) | 0.0375 (0.75% w/v) |

**Example 60.** Comparative example of aqueous composition of triamcinolone hexacetonide (THA, 0.7%) included in hydroxypropyl-beta-cyclodextrin (HPβCD, 30%) in a chitlac matrix (CTL, 0.75%).

The formulation of Example 60 was obtained following the same procedure reported in Example 57, and using the amounts shown in the table below. The pH of the solution was 7.4.

| **#** | **HPβCD (g, % w/v)** | **THA (g, % w/v)** | **CTL (g, % w/v)** |
|---|---|---|---|
| **58** | 1.5 (30% w/v) | 0.035 (0.7% w/v) | 0.0375 (0.75% w/v) |

**Examples 61-63.** Comparative examples of aqueous compositions of triamcinolone acetonide (TA, 0.44%) included in sulfobutylether-7-beta-cyclodextrin (SBECD, 30%) in a matrix of hyaluronic acid alone (HA) at varying concentrations.

1.5 g of sulfobutylether-7-beta-cyclodextrin were dissolved in 5 mL of phosphate buffered saline solution (PBS 1X: 137 mM NaCl, 2.7 mM KCI, 8.1 mM Na₂HPO₄, 1.76 mM NaH₂PO₄), 22 mg of triamcinolone acetonide were subsequently added, and the system thus obtained was stirred for 16 h at room temperature. 37,5, 50, and 62.5 mg of hyaluronic acid were then added under stirring, respectively, and the mixture thus obtained was stirred at 60°C for 2 h and at room temperature for 16 h. The solution has a pH of 7.4.

| **#** | **SBECD (g, % w/v)** | **TA (g, % w/v)** | **HA (g, % w/v)** |
|---|---|---|---|
| **61** | 1.5 (30%) | 0.022 (0.44%) | 0.0375 (0.75%) |
| **62** | 1.5 (30%) | 0.022 (0.44%) | 0.050 (1.0%) |
| **63** | 1.5 (30%) | 0.022 (0.44%) | 0.0625 (1.25%) |

**Example 64.** Comparative example of aqueous composition of triamcinolone acetonide (TA, 1.2%) included in sulfobutylether-7-beta-cyclodextrin (SBECD, 30%) and polysorbate (0.05%) in a matrix of hyaluronic acid alone (HA, 0.75%).

1.5 g of sulfobutylether-7-beta-cyclodextrin and 2.5 mg of polysorbate 20 were dissolved in 5 mL of phosphate buffered saline solution (PBS 1X: 137 mM NaCl, 2.7 mM KCI, 8.1 mM Na₂HPO₄, 1.76 mM NaH₂PO₄), 22 mg of triamcinolone acetonide were subsequently added, and the system thus obtained was stirred for 16 h at room temperature. 37.5 mg of hyaluronic acid were then added under stirring, and the mixture thus obtained was stirred at 60°C for 2 h and at room temperature for 16 h. The pH of the solution was 7.4.

| **#** | **SBECD (g, % w/v)** | **TA (g, % w/v)** | **POLYSORBATE 20 (mg, % w/v)** | **HA (g, % w/v)** |
|---|---|---|---|---|
| **64** | 1.5 (30%) | 0.060 (1.2%) | 2.5 (0.05%) | 0.0375 (0.75%) |

**Examples 65-67.** Comparative examples of aqueous compositions of triamcinolone acetonide (TA, 0.44%) included in hydroxypropyl-beta-cyclodextrin (HPβCD, 30%) in a matrix of hyaluronic acid alone (HA) at varying concentrations.

The formulations of Examples 65-67 were obtained following the procedure reported in Example 61, and using the amounts shown in the table below. The pH of the solutions was 7.4.

| **#** | **HPβCD % w/v)** | **TA (g, % w/v)** | **HA (g, % w/v)** |
|---|---|---|---|
| **65** | 1.5 (30%) | 0.022 (0.44%) | 0.0375 (0.75%) |
| **66** | 1.5 (30%) | 0.022 (0.44%) | 0.050 (1.0%) |
| **67** | 1.5 (30%) | 0.022 (0.44%) | 0.0625 (1.25%) |

**Example 68.** Comparative example of aqueous composition of triamcinolone acetonide (TA, 1.2%) included in hydroxypropyl-beta-cyclodextrin (HPβCD, 30%) and polysorbate (0.05%) in a matrix of hyaluronic acid alone (HA, 0.75%).

The formulation of Example 68 was obtained following the procedure reported in Example 64, and using the amounts shown in the table below. The pH of the solutions was 7.4.

| **#** | **HPβCD (g, % w/v)** | **TA (g, % w/v)** | **POLYSORBATE 20 (mg, % w/v)** | **HA (g, % w/v)** |
|---|---|---|---|---|
| **68** | 1.5 (30%) | 0.060 (1.2%) | 2.5 (0.05%) | 0.0375 (0.75%) |

**Examples 69-71.** Comparative examples of aqueous compositions of triamcinolone hexacetonide (THA, 0.60%) included in sulfobutylether-7-beta-cyclodextrin (SBECD, 30%) in a matrix of hyaluronic acid alone (HA) at varying concentrations. Formulations of Examples 69-71 were obtained following the procedure reported in Example 61, and using the amounts shown in the table below. The pH of the solutions was 7.4.

| **#** | **SBECD (g, % w/v)** | **THA (g, % w/v)** | **HA (g, % w/v)** |
|---|---|---|---|
| **69** | 1.5 (30%) | 0.030 (0.60%) | 0.0375 (0.75%) |
| **70** | 1.5 (30%) | 0.030 (0.60%) | 0.050 (1.0%) |
| **71** | 1.5 (30%) | 0.030 (0.60%) | 0.0625 (1.25%) |

**Examples 72-73.** Comparative examples of aqueous compositions of triamcinolone hexacetonide (THA, 0.60 and 1.2%) included in sulfobutylether-7-beta-cyclodextrin (SBECD, 30%) and polysorbate (0.05%) in a matrix of hyaluronic acid alone (HA, 0.75%).

The formulations of Examples 72-73 were obtained following the procedure reported in Example 64, and using the amounts shown in the table below. The pH of the solutions was 7.4.

| **#** | **SECD (g, % w/v)** | **THA (g, % w/v)** | **POLYSORBATE 20 (mg, % w/v)** | **HA (g, % w/v)** |
|---|---|---|---|---|
| **72** | 1.5 (30%) | 0.030 (0.6%) | 2.5 (0.05%) | 0.0375 (0.75%) |
| **73** | 1.5 (30%) | 0.060 (1.2%) | 2.5 (0.05%) | 0.0375 (0.75%) |

**Examples 74-76.** Comparative examples of aqueous compositions of triamcinolone hexacetonide (THA, 0.70%) included in hydroxypropyl-beta-cyclodextrin (HPβCD, 30%) in a matrix of hyaluronic acid alone (HA) at varying concentrations.

Formulations of Examples 74-76 were obtained following the procedure reported in Example 61, and using the amounts shown in the table below. The pH of the solutions was 7.4.

| **#** | **HPβCD (g, % w/v)** | **THA (g, % w/v)** | **HA (g, % w/v)** |
|---|---|---|---|
| **74** | 1.5 (30%) | 0.035 (0.70%) | 0.0375 (0.75%) |
| **75** | 1.5 (30%) | 0.035 (0.70%) | 0.050 (1.0%) |
| **76** | 1.5 (30%) | 0.035 (0.70%) | 0.0625 (1.25%) |

**Example 77.** Comparative example of aqueous composition of triamcinolone hexacetonide (THA, 1.2%) included in hydroxypropyl-beta-cyclodextrin (HPβCD, 30%) and polysorbate (0.05%) in a matrix of hyaluronic acid alone (HA, 0.75%).

The formulation of Example 77 was obtained following the procedure reported in Example 64, and using the amounts shown in the table below. The pH of the solution was 7.4.

| **#** | **HPβCD (g, % w/v)** | **THA (g, % w/v)** | **POLYSORBATE 20 (mg, % w/v)** | **HA (g, % w/v)** |
|---|---|---|---|---|
| **77** | 1.5 (30%) | 0.060 (1.2%) | 2.5 (0.05%) | 0.0375 (0.75%) |

**Examples 78-79.** Comparative examples of aqueous compositions of diclofenac sodium salt (DCNa, 1.5%) included in sulfobutylether-7-beta-cyclodextrin (SBECD, 5%) in a matrix of hyaluronic acid alone (HA, 0.75 and 1.25%).

Formulations of Examples 78-79 were obtained following the procedure reported in Example 61, and using the amounts shown in the table below. The pH of the solutions was 7.4.

| **#** | **SBECD (g, % w/v)** | **DCNa (g, % w/v)** | **HA (g, % w/v)** |
|---|---|---|---|
| **78** | 0.25 (5%) | 0.075 (1.5%) | 0.0375 (0.75%) |
| **79** | 0.25 (5%) | 0.075 (1.5%) | 0.0625 (1.25%) |

**Example 80.** Comparative example of aqueous composition of diclofenac sodium salt (DCNa, 1.2%) included in sulfobutylether-7-beta-cyclodextrin (SBECD, 5%) and polysorbate (0.05%) in a matrix of hyaluronic acid alone (HA, 0.75%).

The formulation of Example 80 was obtained following the procedure reported in Example 64, and using the amounts shown in the table below. The pH of the solution was 7.4.

| **#** | **SBECD (g, % w/v)** | **DCNa (g, % w/v)** | **POLYSORBATE 20 (mg, % w/v)** | **HA (g, % w/v)** |
|---|---|---|---|---|
| **80** | 1.5 (30%) | 0.060 (1.2%) | 2.5 (0.05%) | 0.0375 (0.75%) |

**Example 81-82.** Comparative examples of aqueous compositions of diclofenac sodium salt (DCNa, 1.5%) included in hydroxypropyl-beta-cyclodextrin (HPβCD, 5%) in a matrix of hyaluronic acid alone (HA, 0.75% and 1.25%).

The formulation of Examples 81-82 was obtained following the procedure reported in Example 61, and using the amounts shown in the table below. The pH of the solutions was 7.4.

| **#** | **HPβCD (g, % w/v)** | **DCNa (g, % w/v)** | **HA (g, % w/v)** |
|---|---|---|---|
| **81** | 0.25 (5%) | 0.075 (1.5%) | 0.0375 (0.75%) |
| **82** | 0.25 (5%) | 0.075 (1.5%) | 0.0625 (1.25%) |

**Example 83.** Comparative example of aqueous composition of diclofenac sodium salt (DCNa, 1.5%) included in hydroxypropyl-beta-cyclodextrin (HPβCD, 5%) and polysorbate (0.05%) in a matrix of hyaluronic acid alone (HA, 1.25%).

The formulation of Example 83 was obtained following the procedure reported in Example 64, and using the amounts shown in the table below. The pH of the solution was 7.4.

| **#** | **HPβCD (g, % w/v)** | **DCNa (g, % w/v)** | **POLYSORBATE 20 (mg, % w/v)** | **HA (g, % w/v)** |
|---|---|---|---|---|
| **83** | 0.25 (5%) | 0.075 (1.5%) | 2.5 (0.05%) | 0.0625 (1.25%) |

**Example 84.** Comparative example of aqueous composition of diclofenac sodium salt (DCNa, 1.5%) included in hydroxypropyl-gamma-cyclodextrin (HPγCD, 5%) in a matrix of hyaluronic acid alone (HA, 0.75%).

The formulation of Example 84 was obtained following the procedure reported in Example 61, and using the amounts shown in the table below. The pH of the solution was 7.4.

| **#** | **HPγCD (g, % w/v)** | **DCNa (g, % w/v)** | **HA (g, % w/v)** |
|---|---|---|---|
| **84** | 0.25 (5%) | 0.075 (1.5%) | 0.0375 (0.75%) |

**Examples 85-86.** Comparative examples of aqueous compositions of triamcinolone acetonide (TA, 0.44 and 0.27%) included in sulfobutylether-7-beta-cyclodextrin (SBECD, 30 and 15%) without polymer matrix.

1.5 g of sulfobutylether-7-beta-cyclodextrin were dissolved in 5 mL of phosphate buffered saline solution (PBS 1X: 137 mM NaCl, 2.7 mM KCI, 8.1 mM Na₂HPO₄, 1.76 mM NaH₂PO₄), 22 and 13.5 mg of triamcinolone acetonide were added, respectively, and the system thus obtained was stirred for 16 h at room temperature. The pH of the solutions was 7.4.

| **#** | **SBECD (g, % w/v)** | **TA (g, % w/v)** |
|---|---|---|
| **85** | 1.5 (30%) | 0.022 (0.44%) |
| **86** | 0.75 (15%) | 0.0135 (0.27%) |

**Example 87.** Comparative example of aqueous composition of triamcinolone acetonide (TA, 0.44%) included in sulfobutylether-7-beta-cyclodextrin (SBECD, 30 and 15%) and polysorbate (0.05%) without polymer matrix.

1.5 g of sulfobutylether-7-beta-cyclodextrin and 2.5 polysorbate 20 were dissolved in 5 mL of phosphate buffered saline solution (PBS 1X: 137 mM NaCl, 2.7 mM KCI, 8.1 mM Na₂HPO₄, 1.76 mM NaH₂PO₄), 22 mg of triamcinolone acetonide were subsequently added, and the system thus obtained was stirred for 16 h at room temperature. The pH of the solution was 7.4.

| **#** | **SBECD (g, % w/v)** | **TA (g, % w/v)** | **POLYSORBATE 20 (mg, % w/v)** |
|---|---|---|---|
| **87** | 1.5 (30%) | 0.022 (0.44%) | 2.5 (0.05%) |

**Examples 88-89.** Comparative examples of aqueous compositions of triamcinolone acetonide (TA, 0.44 and 0.27%) included in hydroxypropyl-beta-cyclodextrin (HPβCD, 30 and 15%) without polymer matrix.

The formulations of Examples 88-89 were obtained following the procedure reported in Examples 85 and 86, and using the amounts shown in the table below. The pH of the solutions was 7.4.

| **#** | **HPβCD (g, % w/v)** | **TA (g, % w/v)** |
|---|---|---|
| **88** | 1.5 (30%) | 0.022 (0.44%) |
| **89** | 0.75 (15%) | 0.0135 (0.27%) |

**Example 90.** Comparative example of aqueous composition of triamcinolone acetonide (TA, 1.2%) included in hydroxypropyl-beta-cyclodextrin (HPβCD, 30%) and polysorbate (0.05%) without polymer matrix.

The formulation of Example 90 was obtained following the procedure reported in Example 87, and using the amounts shown in the table below. The pH of the solution was 7.4.

| **#** | **HPβCD (g, % w/v)** | **TA (g, % w/v)** | **POLYSORBATE 20 (mg, % w/v)** |
|---|---|---|---|
| **90** | 1.5 (30%) | 0.060 (1.2%) | 2.5 (0.05%) |

**Examples 91-92.** Comparative examples of aqueous composition of triamcinolone acetonide (TA, 0.44%) included in sulfobutylether-7-beta-cyclodextrin (SBECD, 30%) and polysorbate (0.06% and 0.04%) without polymer matrix.

The formulations of Examples 91-92 were obtained following the procedure reported in Example 87, and using the amounts shown in the table below. The pH of the solutions was 7.4.

| **#** | **SBECD (g, % w/v)** | **TA (g, % w/v)** | **POLYSORBATE 20 (mg, % w/v)** |
|---|---|---|---|
| **91** | 1.5 (30%) | 0.022 (0.44%) | 2.0 (0.04%) |
| **92** | 1.5 (30%) | 0.022 (0.44%) | 3.0 (0.06%) |

**Examples 93-94.** Comparative examples of aqueous compositions of triamcinolone acetonide (TA, 0,44%) included in sulfobutylether-7-beta-cyclodextrin (SBECD, 30 and 15%), polysorbate (0.04% and 0.06%), and PEG 5000 (9%) without polymer matrix.

1.5 g of sulfobutylether-7-beta-cyclodextrin and 2.0 and 3.0 mg, respectively, of polysorbate 20, 0.45 g of propylene glycol were dissolved in 5 mL of phosphate buffered saline solution (PBS 1X: 137 mM NaCl, 2.7 mM KCI, 8.1 mM Na₂HPO₄, 1.76 mM NaH₂PO₄), 22 mg of triamcinolone acetonide were subsequently added, and the system thus obtained was stirred for 16 h at room temperature. The pH of the solutions was 7.4.

| **#** | **SBECD (g, % w/v)** | **TA (g, % w/v)** | **POLYSORBATE 20 (mg, % w/v)** | **PEG 5000 (g, % w/v)** |
|---|---|---|---|---|
| **93** | 1.5 (30%) | 0.022 (0.44%) | 2.0 (0.04%) | 0.45 (9%) |
| **94** | 1.5 (30%) | 0.022 (0.44%) | 3.0 (0.06%) | 0.45 (9%) |

**Examples 95-96.** Comparative examples of aqueous compositions of triamcinolone acetonide (TA, 0.44%) included in sulfobutylether-7-beta-cyclodextrin (SBECD, 30 and 15%) and Pluronic F68 (0.04% and 0.06%) without polymer matrix.

1.5 g of sulfobutylether-7-beta-cyclodextrin and 2.0 and 3.0 mg, respectively, of Pluronic F68 20 were dissolved in 5 mL of phosphate buffered saline solution (PBS 1X: 137 mM NaCl, 2.7 mM KCI, 8.1 mM Na₂HPO₄, 1.76 mM NaH₂PO₄), 22 mg of triamcinolone acetonide were subsequently added, and the system thus obtained was stirred for 16 h at room temperature. The pH of the solutions was 7.4.

| **#** | **SBECD (g, % w/v)** | **TA (g, % w/v)** | **PLURONIC F68 (mg, % w/v)** |
|---|---|---|---|
| **95** | 1.5 (30%) | 0.022 (0.44%) | 2.0 (0.04%) |
| **96** | 1.5 (30%) | 0.022 (0.44%) | 3.0 (0.06%) |

**Examples 97-98.** Comparative examples of aqueous compositions of triamcinolone acetonide (TA, 0.44%) included in sulfobutylether-7-beta-cyclodextrin (SBECD, 30 and 15%), Pluronic F68 (0.04% and 0.06%), and PEG 5000 (9%) without polymer matrix.

1.5 g of sulfobutylether-7-beta-cyclodextrin and 2.0 and 3.0 mg, respectively, of Pluronic F68 20, 0.45 g of propylene glycol were dissolved in 5 mL of phosphate buffered saline solution (PBS 1X: 137 mM NaCl, 2.7 mM KCI, 8.1 mM Na₂HPO₄, 1.76 mM NaH₂PO₄), 22 mg of triamcinolone acetonide were subsequently added, and the system thus obtained was stirred for 16 h at room temperature. The pH of the solutions was 7.4.

| **#** | **SBECD (g, % w/v)** | **TA (g, % w/v)** | **PLURONIC F68 (mg, % w/v)** | **PEG 5000 (g, % w/v)** |
|---|---|---|---|---|
| **97** | 1.5 (30%) | 0.022 (0.44%) | 2.0 (0.04%) | 0.45 (9%) |
| **98** | 1.5 (30%) | 0.022 (0.44%) | 3.0 (0.06%) | 0.45 (9%) |

**Examples 99-100.** Comparative examples of aqueous compositions of triamcinolone hexacetonide (THA, 0.60 and 0.16%) included in sulfobutylether-7-beta-cyclodextrin (SBECD, 30%) without polymer matrix.

The formulations of Examples 99-100 were obtained following the procedure reported in Example 85, and using the amounts shown in the table below. The pH of the solutions was 7.4.

| **#** | **SBECD (g, % w/v)** | **THA (g, % w/v)** |
|---|---|---|
| **99** | 1.5 (30%) | 0.030 (0.60%) |
| **100** | 0.75 (15%) | 0.008 (0.16%) |

**Examples 101-102.** Comparative examples of aqueous compositions of triamcinolone hexacetonide (THA, 0.60 and 1.2%) included in sulfobutylether-7-beta-cyclodextrin (SBECD, 30%) and polysorbate (0.05%) without polymer matrix. The formulations of Examples 101-102 were obtained following the procedure reported in Example 87, and using the amounts shown in the table below. The pH of the solutions was 7.4.

| **#** | **SBECD (g, % w/v)** | **THA (g, % w/v)** | **POLYSORBATE 20 (mg, % w/v)** |
|---|---|---|---|
| **101** | 1.5 (30%) | 0.030 (0.60%) | 2.5 (0.05%) |
| **102** | 1.5 (30%) | 0.060 (1.2%) | 2.5 (0.05%) |

**Example 103.** Comparative example of aqueous composition of triamcinolone hexacetonide (THA, 0.70%) included in hydroxypropyl-beta-cyclodextrin (HPβCD, 30%) without polymer matrix.

The formulation of Example 103 was obtained following the procedure reported in Example 85, and using the amounts shown in the table below. The pH of the solution was 7.4.

| **#** | **HPβCD (g, % w/v)** | **THA (g, % w/v)** |
|---|---|---|
| **103** | 1.5 (30%) | 0.035 (0.70%) |

**Example 104.** Comparative example of aqueous composition of triamcinolone hexacetonide (THA, 1.2%) included in hydroxypropyl-beta-cyclodextrin (HPβCD, 30%) and polysorbate (0.05%) without polymer matrix.

The formulation of Example 104 was obtained following the procedure reported in Example 87, and using the amounts shown in the table below. The pH of the solution was 7.4.

| **#** | **HPβCD (g, % w/v)** | **THA (g% w/v)** | **POLYSORBATE 20 (mg, % w/v)** |
|---|---|---|---|
| **104** | 1.5 (30%) | 0.060 (1.2%) | 2.5 (0.05%) |

**Example 105.** Comparative example of aqueous composition of diclofenac sodium salt (DCNa, 1.5%) included in sulfobutylether-7-beta-cyclodextrin (SBECD, 5%) without polymer matrix.

The formulation of Example 105 was obtained following the procedure reported in Example 85, and using the amounts shown in the table below. The pH of the solution was 7.4.

| **#** | **SBECD (g, % w/v)** | **DCNa (g, % w/v)** |
|---|---|---|
| **105** | 0.25 (5%) | 0.075 (1.5%) |

**Example 106.** Comparative example of aqueous composition of diclofenac sodium salt (DCNa, 1.5%) included in hydroxypropyl-beta-cyclodextrin (HPβCD, 5%) without polymer matrix.

The formulation of Example 106 was obtained following the procedure reported in Example 85, and using the amounts shown in the table below. The pH of the solution was 7.4.

| **#** | **HPβCD (g, % w/v)** | **DCNa (g, % w/v)** |
|---|---|---|
| **106** | 0.25 (5%) | 0.075 (1.5%) |

**Example 107.** Comparative example of aqueous composition of diclofenac sodium salt (DCNa, 1.5%) included in hydroxypropyl-gamma-cyclodextrin (HPγCD, 5%) without polymer matrix.

The formulation of Example 107 was obtained following the procedure reported in Example 85, and using the amounts shown in the table below. The pH of the solution was 7.4.

| **#** | **HPγCD (g, % w/v)** | **DCNa (g, % w/v)** |
|---|---|---|
| **107** | 0.25 (5%) | 0.075 (1.5%) |

### Examples 108-110

Aqueous composition of piroxicam (PYR, 0.05 %) included in various cyclodextrins (5 %) in a matrix of hyaluronic acid and chitlac 0.75 % and 0.5 %, respectively.

The formulations of Examples 108-110 were obtained following the procedure reported in Example 1, and using the amounts shown in the table below. The pH of the solution was 7.4.

| **#** | **PYR (g, % w/v)** | **CD** | **CD (g, % w/v)** | **V (mL) PBS 1X** | **V (mL) CTL 2% (w/v) in PBS1X** | **HA (g, % w/v)** | **CTL (g, % w/v)** |
|---|---|---|---|---|---|---|---|
| **108** | 0.0025 (0.05 %) | SBECD | 0.25 (5 %) | 3.75 | 1.25 | 0.0375 (0.75 %) | 0.025 (0.50 %) |
| **109** | 0.0025 (0.05 %) | **HPβCD** | 0.25 (5 %) | 3.75 | 1.25 | 0.0375 (0.75 %) | 0.025 (0.50 %) |
| **110** | 0.0025 (0.05 %) | HPγCD | 0.25 (5 %) | 3.75 | 1.25 | 0.0375 (0.75 %) | 0.025 (0.50 %) |

### Examples 111-113

Aqueous composition of ketorolac (KET, 0.25 %) included in various cyclodextrins (5 %) in a matrix of hyaluronic acid and chitlac 0.75 % and 0.5 %, respectively.

The formulations of Examples 111-113 were obtained following the procedure reported in Example 1, and using the amounts shown in the table below. The pH of the solution was 7.4.

| **#** | **KET (g, % w/v)** | **CD** | **CD (g, % w/v)** | **V (mL) PBS 1X** | **V (mL) CTL 2% (w/v) in PBS1X** | **HA (g, % w/v)** | **CTL (g, % w/v)** |
|---|---|---|---|---|---|---|---|
| **111** | 0.0125 (0.25 %) | SBECD | 0.25 (5 %) | 3.75 | 1.25 | 0.0375 (0.75 %) | 0.025 (0.50 %) |
| **112** | 0.0125 (0.25 %) | **HPβCD** | 0.25 (5 %) | 3.75 | 1.25 | 0.0375 (0.75 %) | 0.025 (0.50 %) |
| **113** | 0.0125 (0.25 %) | HPγCD | 0.25 (5 %) | 3.75 | 1.25 | 0.0375 (0.75 %) | 0.025 (0.50 %) |

### Examples 114-116

Comparative examples of aqueous composition of piroxicam (PYR, 0.05 %) included in various cyclodextrins (5 %) in a matrix of hyaluronic acid 0.75 %.

The formulations of Examples 114-117 were obtained following the procedure reported in Example 61, and using the amounts shown in the table below. The pH of the solution was 7.4.

| **#** | **PYR (g, % w/v)** | **CD** | **CD (g, % w/v)** | **HA (g, % w/v)** |
|---|---|---|---|---|
| **114** | 0.0025 (0.05 %) | SBECD | 0.25 (5 %) | 0.0375 (0.75 %) |
| **115** | 0.0025 (0.05 %) | **HPβCD** | 0.25 (5 %) | 0.0375 (0.75 %) |
| **116** | 0.0025 (0.05 %) | HPγCD | 0.25 (5 %) | 0.0375 (0.75 %) |

### Examples 117-119

Comparative examples of aqueous composition of ketorolac (KET, 0.25 %) included in various cyclodextrins (5 %) in a matrix of hyaluronic acid 0.75 %.

The formulations of Examples 117-119 were obtained following the procedure reported in Example 61, and using the amounts shown in the table below. The pH of the solution was 7.4.

| **#** | **KET (g, % w/v)** | **CD** | **CD (g, % w/v)** | **HA (g, % w/v)** |
|---|---|---|---|---|
| **117** | 0.0125 (0.25 %) | SBECD | 0.25 (5 %) | 0.0375 (0.75 %) |
| **118** | 0.0125 (0.25 %) | HPβCD | 0.25 (5 %) | 0.0375 (0.75 %) |
| **119** | 0.0125 (0.25 %) | HPγCD | 0.25 (5 %) | 0.0375 (0.75 %) |

### Examples 120-122

Comparative examples of aqueous composition of piroxicam (PYR, 0.05 %) included in various cyclodextrins (5 %) without polymer matrix.

The formulations of Examples 120-122 were obtained following the procedure reported in Example 85, and using the amounts shown in the table below. The pH of the solution was 7.4.

| **#** | **PYR (g, % w/v)** | **CD** | **CD (g, % w/v)** |
|---|---|---|---|
| **120** | 0.0025 (0.05 %) | SBECD | 0.25 (5 %) |
| **121** | 0.0025 (0.05 %) | HPβCD | 0.25 (5 %) |
| **122** | 0.0025 (0.05 %) | HPγCD | 0.25 (5 %) |

### Examples 123-125

Comparative examples of aqueous composition of ketorolac (KET, 0.25 %) included in various cyclodextrins (5 %) without polymer matrix.

The formulations of Examples 123-125 were obtained following the procedure reported in Example 85, and using the amounts shown in the table below. The pH of the solution was 7.4.

| **#** | **KET (g, % w/v)** | **CD** | **CD (g, % w/v)** |
|---|---|---|---|
| **123** | 0.0125 (0.25 %) | SBECD | 0.25 (5 %) |
| **124** | 0.0125 (0.25 %) | HPβCD | 0.25 (5 %) |
| **125** | 0.0125 (0.25 %) | HPγCD | 0.25 (5 %) |

The compositions obtained according to Examples 1-125 were tested for:
- effect of the polysaccharide polymer matrix on solubilization of the active ingredient included in the cyclodextrin;
- effect of additional components on solubilization of the active ingredient included in the cyclodextrin;
- effect of the polysaccharide polymer matrix on the active ingredient release kinetics.

**Example 126.** Effect of the polysaccharide polymer matrix on the solubilizing capacity of various cyclodextrins with water-insoluble active ingredients: triamcinolone acetonide (TA) and triamcinolone hexacetonide (THA).

Table 1 shows selected triamcinolone solubility data in the presence of polysaccharide components and cyclodextrins (a = percentage value based on a API@CD system without polymers, calculated as 100*(API@CD@POLY/API@CD).

**Table 1**

| **#** | **API** | **API solubilized (%^{a})** | **CD** | **CD (%)** | **HA (%)** | **CTL (%)** |
|---|---|---|---|---|---|---|
| **85** | TA | 100 | SBECD | 30 | 0 | 0 |
| **62** | TA | 90 | SBECD | 30 | 1 | 0 |
| **5** | TA | 95 | SBECD | 30 | 1 | 0.25 |
| **3** | TA | 89 | SBECD | 30 | 1 | 0.75 |
| **57** | TA | 93 | SBECD | 30 | 0 | 0.75 |
| **1** | TA | 88 | SBECD | 30 | 0.25 | 0.75 |
| **2** | TA | 95 | SBECD | 30 | 0.75 | 0.75 |
| **88** | TA | 100 | HPβCD | 30 | 0 | 0 |
| **66** | TA | 105 | HPβCD | 30 | 1 | 0 |
| **17** | TA | 107 | HPβCD | 30 | 1 | 0.25 |
| **15** | TA | 104 | HPβCD | 30 | 1 | 0.75 |
| **58** | TA | 108 | HPβCD | 30 | 0 | 0.75 |
| **13** | TA | 118 | HPβCD | 30 | 0.25 | 0.75 |
| **14** | TA | 118 | HPβCD | 30 | 0.75 | 0.75 |
| **99** | THA | 100 | SBECD | 30 | 0 | 0 |
| **70** | THA | 106 | SBECD | 30 | 1 | 0 |
| **29** | THA | 110 | SBECD | 30 | 1 | 0.25 |
| **27** | THA | 98 | SBECD | 30 | 1 | 0.75 |
| **59** | THA | 114 | SBECD | 30 | 0 | 0.75 |
| **25** | THA | 105 | SBECD | 30 | 0.25 | 0.75 |
| **26** | THA | 113 | SBECD | 30 | 0.75 | 0.75 |
| **103** | THA | 100 | HPβCD | 30 | 0 | 0 |
| **75** | THA | 100 | HPβCD | 30 | 1 | 0 |
| **42** | THA | 90 | HPβCD | 30 | 1 | 0.25 |
| **40** | THA | 67 | HPβCD | 30 | 1 | 0.75 |
| **60** | THA | 107 | HPβCD | 30 | 0 | 0.75 |
| **38** | THA | 90 | HPβCD | 30 | 0.25 | 0.75 |
| **39** | THA | 107 | HPβCD | 30 | 0.75 | 0.75 |

As can be seen from the above results, variations in the active ingredient solubility were observed for the composition based on cyclodextrin and hyaluronic acid. The same can be observed when the cyclodextrin-active ingredient system is added with either chitlac or chitlac and hyaluronic acid.

**Example 127.** Effect of cyclodextrin SBECD with triamcinolone acetonide (TA) in association with polysorbates, polyethylene glycol and poloxamers on the solubilizing capacity.

For comparison purposes, the effect of other excipients commonly used in pharmaceutical compositions, such as polysorbates, polyethylene glycol and poloxamers, on the cyclodextrin-active ingredient solubility was investigated.

The compositions were prepared as described in the examples.

In most cases no variations were observed (Table 2), in other cases there were modest and, in any case, always positive variations, thus enhancing the solubility of the inclusion complex, and therefore confirming that these excipients may be used without any preclusion.

**Table 2**

| **#** | **API** | **API solubilized** | **CD** | **CD** | **Tween 20** | **Pluronic F68** | **PEG 5000** |
|---|---|---|---|---|---|---|---|
| | | **(%^{a})** | | **(%)** | **(%)** | **(%)** | **(%)** |
| **85** | TA | 100 | SBECD | 30 | 0 | 0 | 0 |
| **91** | TA | 100 | SBECD | 30 | 0.04 | 0 | 0 |
| **92** | TA | 103 | SBECD | 30 | 0.06 | 0 | 0 |
| **95** | TA | 106 | SBECD | 30 | 0 | 0.04 | 0 |
| **96** | TA | 100 | SBECD | 30 | 0 | 0.06 | 0 |
| **93** | TA | 100 | SBECD | 30 | 0.04 | 0 | 9 |
| **94** | TA | 107 | SBECD | 30 | 0.06 | 0 | 9 |
| **97** | TA | 112 | SBECD | 30 | 0 | 0.04 | 9 |
| **98** | TA | 114 | SBECD | 30 | 0 | 0.06 | 9 |

| | | | | | | | |
|---|---|---|---|---|---|---|---|
| (a = percentage value based on a API@CD system without polymers, calculated as 100*(API@CD@ECCIP/API@CD). | | | | | | | |

**Example 128.** Release kinetics of triamcinolone acetonide (TA), triamcinolone hexacetonide (THA), and diclofenac, piroxicam and ketorolac included in SBECD, HPβCD, and HPγCD cyclodextrins from the polysaccharide polymer matrix formed by hyaluronic acid (HA) and chitlac (CTL)

0.500 g of composition were transferred into a well (Slide-A-Lyzer mini dialysis device, 10k-MWCO, product code: 69570, Thermo Fisher Scientific) equipped with a dialysis membrane on the bottom (cut-off 10 KDa), previously treated with deionized water for 30 minutes. The well was then sealed and immersed in 5 mL of saline phosphate buffer (PBS1X: NaCl 137 mM, KCI 2.7 mM, Na₂HPO₄ 8.1 mM, NaH₂PO₄ 1.76 mM) added with 2.5 mg of polysorbate 20 (0.05%). After the desired amount of time, the concentration of the active ingredient retained in the well was quantified by UV-Vis.

The results obtained after 24 hours are shown in the Table 3 below, while Figure 1 shows, by way of example, a typical release profile of the systems in question.

**Table 3**

| **#** | **API** | **CD** | **CD (% w/v)** | **HA (% w/v)** | **CTL (% w/v)** | **release@24 h (%)** |
|---|---|---|---|---|---|---|
| **85** | TA | SBECD | 30 | 0 | 0 | 72 |
| **61** | TA | SBECD | 30 | 0.75 | 0 | 63 |
| **4** | TA | SBECD | 30 | 0.75 | 0.50 | 50 |
| **88** | TA | HPβCD | 30 | 0 | 0 | 59 |
| **65** | TA | HPβCD | 30 | 0.75 | 0 | 52 |
| **16** | TA | HPβCD | 30 | 0.75 | 0.50 | 44 |
| **99** | THA | SBECD | 30 | 0 | 0 | 87 |
| **69** | THA | SBECD | 30 | 0.75 | 0 | 78 |
| **28** | THA | SBECD | 30 | 0.75 | 0.5 | 64 |
| **103** | THA | HPβCD | 30 | 0 | 0 | 59 |
| **74** | THA | HPβCD | 30 | 0.75 | 0 | 46 |
| **41** | THA | HPβCD | 30 | 0.75 | 0.5 | 41 |
| **89** | TA | HPβCD | 15 | 0 | 0 | 64 |
| **20** | TA | HPβCD | 15 | 0.75 | 0.5 | 57 |
| **18** | TA | HPβCD | 30 | 1.14 | 0.11 | 49 |
| **17** | TA | HPβCD | 30 | 1.0 | 0.25 | 55 |
| **33** | THA | SBECD | 30 | 0.75 | 0.5 | 85 |
| **19** | TA | HPβCD | 30 | 1.2 | 0.8 | 53 |
| **105** | DCNa | SBECD | 5 | 0 | 0 | 75 |
| **78** | DCNa | SBECD | 5 | 0.75 | 0 | 69 |
| **50** | DCNa | SBECD | 5 | 0.75 | 0.5 | 65 |
| **106** | DCNa | HPβCD | 5 | 0 | 0 | 57 |
| **81** | DCNa | HPβCD | 5 | 0.75 | 0 | 57 |
| **53** | DCNa | HPβCD | 5 | 0.75 | 0.5 | 41 |
| **107** | DCNa | HPγCD | 5 | 0 | 0 | 49 |
| **84** | DCNa | HPγCD | 5 | 0.75 | 0 | 46 |
| **56** | DCNa | HPγCD | 5 | 0.75 | 0.5 | 9 |
| **120** | PYR | SBECD | 5 | 0 | 0 | 57 |
| **114** | PYR | SBECD | 5 | 0.75 | 0 | 51 |
| **108** | PYR | SBECD | 5 | 0.75 | 0.5 | 47 |
| **121** | PYR | HPβCD | 5 | 0 | 0 | 45 |
| **115** | PYR | HPβCD | 5 | 0.75 | 0 | 44 |
| **109** | PYR | HPβCD | 5 | 0.75 | 0.5 | 36 |
| **122** | PYR | HPγCD | 5 | 0 | 0 | 62 |
| **116** | PYR | HPγCD | 5 | 0.75 | 0 | 46 |
| **110** | PYR | HPγCD | 5 | 0.75 | 0.5 | 35 |
| **123** | KET | SBECD | 5 | 0 | 0 | 40 |
| **117** | KET | SBECD | 5 | 0.75 | 0 | 26 |
| **111** | KET | SBECD | 5 | 0.75 | 0.5 | 24 |
| **124** | KET | HPβCD | 5 | 0 | 0 | 27 |
| **118** | KET | HPβCD | 5 | 0.75 | 0 | 20 |
| **112** | KET | HPβCD | 5 | 0.75 | 0.5 | 16 |
| **125** | KET | HPγCD | 5 | 0 | 0 | 43 |
| **119** | KET | HPγCD | 5 | 0.75 | 0 | 40 |
| **113** | KET | HPγCD | 5 | 0.75 | 0.5 | 36 |

The analysis of the data listed in Table 3 shows that the active ingredient release is increasingly rapid when the system is deprived of the polysaccharide matrix, the introduction of a single polymer component, such as hyaluronic acid, in the system allows to slow down the release rate, and a further slowdown of the active ingredient release occurs when chitlac is present in the polysaccharide matrix.

In conclusion, the type of cyclodextrin chosen in the specific formulation allows to determine the degree of release and, more precisely, it has been observed that SBECD allows to achieve higher release values, faster than HPβCD, in terms of total amount of released API.

### Example 129

Comparative examples of percent release at 24 hours of triamcinolone acetonide (TA), triamcinolone hexacetonide (THA) diclofenac, piroxicam and ketorolac included in SBECD, HPβCD, and HPγCD cyclodextrins from the polysaccharide polymer matrix formed by hyaluronic acid (HA) and chitlac (CTL) normalized to the percent release at 24 hours of triamcinolone acetonide (TA), triamcinolone hexacetonide (THA), diclofenac, piroxicam and ketorolac included in SBECD, HPβCD, and HPγCD cyclodextrins without polymer matrix.

| **#** | **API** | **CD** | **Normalized release@24 h (%)** |
|---|---|---|---|
| **4** | TA | SBECD | 69 |
| **16** | TA | HPβCD | 75 |
| **28** | THA | SBECD | 74 |
| **41** | THA | HPβCD | 69 |
| **50** | DCNa | SBECD | 87 |
| **53** | DCNa | HPβCD | 72 |
| **56** | DCNa | HPγCD | 18 |
| **108** | PYR | SBECD | 82 |
| **109** | PYR | HPβCD | 80 |
| **110** | PYR | HPγCD | 56 |
| **111** | KET | SBECD | 60 |
| **112** | KET | HPβCD | 59 |
| **113** | KET | HPγCD | 84 |

## Claims

1. A composition comprising a clathrate consisting of a cyclodextrin and an active ingredient, wherein the cyclodextrin and active ingredient clathrate is homogeneously dispersed in a polysaccharide polymer matrix in aqueous solution formed by hyaluronic acid and/or salts thereof and an oligosaccharide derivative of chitosan with lactose, obtained by reductive amination reaction of chitosan D-glucosamine, having a degree of substitution of the amine functional group with lactose of at least 40%.

2. The composition according to claim 1, wherein the cyclodextrins are selected from β-cyclodextrin and γ-cyclodextrin ether derivatives and mixtures thereof, wherein one or more hydroxyl groups are substituted with C₁₋₆-alkyl, hydroxy-C_{1- 6}-alkyl, carboxy-C₁₋₆-alkyl, C₁₋₆alkyloxycarbonyl, and C₁₋₄-sulfoalkyl groups.

3. The composition according to claim 2, wherein the cyclodextrins are selected from 2,6-dimethyl-β-cyclodextrin, 2-hydroxyethyl-β-cyclodextrin, 2-hydroxypropyl-β-cyclodextrin, (2-carboxymethoxy)propyl-β-cyclodextrin, 2-hydroxypropyl-γ-cyclodextrin, and sulfobutylether(7)-β-cyclodextrin.

4. The composition according to claim 1, wherein the hyaluronic acid and/or salts thereof have an average molecular weight of between 500 and 10,000 kDa, as measured by gel permeation chromatography.

5. The composition according to claim 4, wherein the hyaluronic acid and/or salts thereof have an average molecular weight of between 500 and 2,000 kDa, as measured by gel permeation chromatography.

6. The composition according to claim 1, wherein the oligosaccharide derivative of chitosan with lactose has an average molecular weight of between 500 and 1,000 kDa, as measured by gel permeation chromatography, and a degree of residual acetylation of between 10 and 20%.

7. The composition according to claim 1, wherein the oligosaccharide derivative of chitosan with lactose has a degree of substitution of between 50 and 70%.

8. The composition according to claim 1, wherein the cyclodextrin is between 1% and 30% w/v.

9. The composition according to claim 1, wherein the polysaccharide matrix formed by hyaluronic acid and/or salts thereof and an oligosaccharide derivative of chitosan with lactose is between 0.5% and 4% w/v.

10. The composition according to claim 9, wherein the single polysaccharide components are between 0.25% and 2% w/v.

11. The composition according to claim 10, wherein the weight ratios of hyaluronic acid and/or salts thereof to the oligosaccharide derivative of chitosan with lactose are between 1:3 and 10:1 (hyaluronic acid:oligosaccharide derivative of chitosan with lactose).

12. The composition according to claim 1, wherein the active ingredients are selected from anti-infectives, and corticosteroids and non-steroidal anti-inflammatory agents.

13. The composition according to claim 12, wherein the active ingredients are selected from the group consisting of triamcinolone acetonide, triamcinolone hexacetonide, diclofenac, piroxicam, ketorolac and mixtures thereof.

14. The composition according to any one of claims 1 to 12 for use in the loco-regional treatment of musculoskeletal diseases, **characterized by** acute and chronic inflammatory conditions.

15. The composition according to claim 14, wherein the musculoskeletal diseases are acute and chronic osteoarticular diseases.

## Patentansprüche

1. Zusammensetzung, die ein Clathrat umfasst, das aus einem Cyclodextrin und einem Wirkstoff besteht, wobei das Clathrat aus Cyclodextrin und Wirkstoff homogen in einer Polysaccharid-Polymermatrix in wässriger Lösung dispergiert ist, die durch Hyaluronsäure und/oder Salzen davon und ein Oligosaccharid-Derivat von Chitosan mit Laktose gebildet ist, erhalten durch reduktive Aminierungsreaktion von Chitosan-D-Glucosamin, mit einem Grad an Substitution der funktionellen Amingruppe mit Laktose von zumindest 40 %.

2. Zusammensetzung nach Anspruch 1, wobei die Cyclodextrine ausgewählt sind aus β-Cyclodextrin- und γ-Cyclodextrinether-Derivaten und Mischungen davon, wobei eine oder mehrere Hydroxylgruppen mit C₁₋₆-Alkyl-, Hydroxy-C₁₋₆-alkyl-, Carboxy-C₁₋₆-alkyl-, C₁₋₆-Alkyloxycarbonyl- und C₁₋₄-Sulfoalkylgruppen substituiert sind.

3. Zusammensetzung nach Anspruch 2, wobei die Cyclodextrine ausgewählt sind aus 2,6-Dimethyl-(3-cyclodextrin, 2-Hydroxyethyl-(3-cyclodextrin, 2-Hydroxypropyl-(3-cyclodextrin, (2-Carboxymethoxy)propyl-(3-cyclodextrin, 2-Hydroxypropyl-γ-cyclodextrin und Sulfobutylether(7)-(3-cyclodextrin.

4. Zusammensetzung nach Anspruch 1, wobei die Hyaluronsäure und/oder Salze davon ein durchschnittliches Molekulargewicht zwischen 500 und 10.000 kDa wie gemessen durch Gelpermeationschromatographie aufweisen.

5. Zusammensetzung nach Anspruch 4, wobei die Hyaluronsäure und/oder Salze davon ein durchschnittliches Molekulargewicht zwischen 500 und 2.000 kDa wie gemessen durch Gelpermeationschromatographie aufweisen.

6. Zusammensetzung nach Anspruch 1, wobei das Oligosaccharid-Derivat von Chitosan mit Laktose ein durchschnittliches Molekulargewicht zwischen 500 und 1.000 kDa wie gemessen durch Gelpermeationschromatographie, und einen Grad an Restacetylierung zwischen 10 und 20 % aufweist.

7. Zusammensetzung nach Anspruch 1, wobei das Oligosaccharid-Derivat von Chitosan mit Lactose einen Grad an Substitution zwischen 50 und 70 % aufweist.

8. Zusammensetzung nach Anspruch 1, wobei das Cyclodextrin zwischen 1 % und 30 % w/v ist.

9. Zusammensetzung nach Anspruch 1, wobei die Polysaccharidmatrix, die durch Hyaluronsäure und/oder Salzen davon und ein Oligosaccharid-Derivat von Chitosan mit Laktose gebildet ist, zwischen 0,5 % und 4 % w/v ist.

10. Zusammensetzung nach Anspruch 9, wobei die einzelnen Polysaccharidkomponenten zwischen 0,25 % und 2 % w/v sind.

11. Zusammensetzung nach Anspruch 10, wobei die Gewichtsverhältnisse von Hyaluronsäure und/oder Salzen davon zu dem Oligosaccharid-Derivat von Chitosan mit Laktose zwischen 1:3 und 10: 1 (Hyaluronsäure:Oligosaccharid-Derivat von Chitosan mit Laktose) sind.

12. Zusammensetzung nach Anspruch 1, wobei die Wirkstoffe ausgewählt sind aus Antiinfektiva und Kortikosteroiden und nichtsteroidalen entzündungshemmenden Mitteln.

13. Zusammensetzung nach Anspruch 12, wobei die Wirkstoffe ausgewählt sind aus der Gruppe bestehend aus Triamcinolonacetonid, Triamcinolonhexacetonid, Diclofenac, Piroxicam, Ketorolac und Mischungen davon.

14. Zusammensetzung nach einem der Ansprüche 1 bis 12 zur Verwendung bei der lokoregionalen Behandlung von Muskel-Skelett-Erkrankungen, die durch akute und chronische Entzündungszustände gekennzeichnet sind.

15. Zusammensetzung nach Anspruch 14, wobei die Muskel-Skelett-Erkrankungen akute und chronische osteoartikuläre Erkrankungen sind.

## Revendications

1. Composition comprenant un clathrate constitué d'une cyclodextrine et d'un principe actif, ladite cyclodextrine et ledit principe actif clathrate étant dispersés de façon homogène dans une matrice polymère de polysaccharide dans une solution aqueuse formée par de l'acide hyaluronique et/ou des sels de celui-ci et un dérivé oligosaccharidique de chitosane avec du lactose, obtenu par une réaction d'amination réductrice de la D-glucosamine chitosane, présentant un degré de substitution du groupe fonctionnel amine par le lactose d'au moins 40 %.

2. Composition selon la revendication 1, lesdites cyclodextrines étant choisies parmi les dérivés d'éthers de β-cyclodextrine et de γ-cyclodextrine et leurs mélanges, un ou plusieurs groupes hydroxyle étant substitués par les groupes C₁₋₆-alkyle, hydroxy-C₁₋₆-alkyle, carboxy-C₁₋₆-alkyle, C₁₋₆-alkyloxycarbonyle et C₁₋₄-sulfoalkyle.

3. Composition selon la revendication 2, lesdites cyclodextrines étant choisies parmi la 2,6-diméthyl-β-cyclodextrine, la 2-hydroxyéthyl-β-cyclodextrine, la 2-hydropropyl-β-cyclodextrine, la (2-carboxyméthoxy)propyl-β-cyclodextrine, la 2-hydropropyl-γ-cyclodextrine, et la sulfobutyléther(7)-β-cyclodextrine.

4. Composition selon la revendication 1, ledit acide hyaluronique et/ou lesdits sels de celui-ci présentant un poids moléculaire moyen compris entre 500 et 10 000 kDa, tel que mesuré par chromatographie par perméation de gel.

5. Composition selon la revendication 4, ledit acide hyaluronique et/ou lesdits sels de celui-ci présentant un poids moléculaire moyen compris entre 500 et 2 000 kDa, tel que mesuré par chromatographie par perméation de gel.

6. Composition selon la revendication 1, ledit dérivé oligosaccharidique de chitosane avec du lactose présentant un poids moléculaire moyen compris entre 500 et 1 000 kDa, tel que mesuré par chromatographie par perméation de gel, et un degré d'acétylation résiduelle compris entre 10 et 20 %.

7. Composition selon la revendication 1, ledit dérivé oligosaccharidique de chitosane avec du lactose présentant un degré de substitution compris entre 50 et 70 %.

8. Composition selon la revendication 1, ladite cyclodextrine représentant entre 1 % et 30 % en p/v.

9. Composition selon la revendication 1, ladite matrice de polysaccharide formée par de l'acide hyaluronique et/ou des sels de celui-ci et un dérivé oligosaccharidique de chitosane avec du lactose représentant entre 0,5 % et 4 % en p/v.

10. Composition selon la revendication 9, lesdits simples composants de polysaccharide représentant entre 0,25 % et 2 % en p/v.

11. Composition selon la revendication 10, les rapports pondéraux de l'acide hyaluronique et/ou des sels de celui-ci au dérivé oligosaccharidique de chitosane avec du lactose étant compris entre 1:3 et 10:1 (acide hyaluronique:dérivé oligosaccharidique de chitosane avec du lactose).

12. Composition selon la revendication 1, lesdits principes actifs étant choisis parmi les anti-infectieux, et les corticostéroïdes et les agents anti-inflammatoires non stéroïdiens.

13. Composition selon la revendication 12, lesdits principes actifs étant choisis dans le groupe constitué par l'acétonide de triamcinolone, l'hexacétonide de triamcinolone, le diclofénac, le piroxicam, le kétorolac et leurs mélanges.

14. Composition selon l'une quelconque des revendications 1 à 12 destinée à être utilisée dans le traitement loco-régional de maladies musculosquelettiques, **caractérisées par** des états inflammatoires aigus et chroniques.

15. Composition selon la revendication 14, lesdites maladies musculosquelettiques étant des maladies ostéoarticulaires aiguës et chroniques.
